# EUROPÄISCHE PATENTSCHRIFT

(11) **EP 2 831 055 B1**
(45) Veröffentlichungstag und Bekanntmachung des Hinweises auf die Patenterteilung: **05.10.2016**
(21) Anmeldenummer: 13713176.9
(22) Anmeldetag: 28.03.2013
(51) Int. Cl.: C07D 317/38, C08F 24/00

(54) **POLYMERISIERBARE ALKYLIDEN-1,3-DIOXOLAN-2-ONE UND DEREN VERWENDUNG**
POLYMERIZABLE ALKYLIDENE-1,3-DIOXOLAN-2-ONES AND USE THEREOF
MONOMÈRES AKLYLIDÈNE-1,3-DIOXOLAN-2-ONE POLYMÉRISABLES ET UTILISATION DESDITS MONOMÈRES

(30) Priorität: 29.03.2012 EP 12162115
(43) Veröffentlichungstag der Anmeldung: 04.02.2015
(73) Patentinhaber: BASF SE, 67056 Ludwigshafen am Rhein (DE)
(72) Erfinder: PORTA GARCIA, Marta, 68165 Mannheim (DE); WEIS, Martine, 67061 Ludwigshafen (DE); LANVER, Andreas, 68199 Mannheim (DE); BLANCHOT, Mathieu, 67065 Ludwigshafen (DE); FLORES-FIGUEROA, Aaron, 68161 Mannheim (DE); KLOPSCH, Rainer, 67551 Worms (DE); HAAF-KLEINHUBBERT, Christina, 69502 Hemsbach (DE); KUTZKI, Olaf, 68163 Mannheim (DE)
(86) Internationale Anmeldenummer: PCT/EP2013/056716
(87) Internationale Veröffentlichungsnummer: WO 2013/144299

(56) Entgegenhaltungen:
- WO-A1-97/23516
- WO-A1-03/048215
- WO-A1-2011/157671
- JP-A- 2006 052 244
- US-A- 3 082 216

## Beschreibung

Die vorliegende Erfindung betrifft polymerisierbare Alkyliden-1,3-dioxolan-2-on-Monomere, deren Herstellung und deren Verwendung zur Herstellung von Polymeren. Die Erfindung betrifft auch die durch Homo- oder Copolymerisation von Alkyliden-1,3-dioxolan-2-on-Monomeren erhältlichen Homo- und Copolymere und deren Verwendung als Komponente in 2K-Bindemittelzusammensetzungen.

Polyurethane (PUR) finden auf unzähligen Gebieten Verwendung, wie beispielsweise der Herstellung von Schäumen, Lacken, Beschichtungen und Klebstoffen. Allen Polyurethanen gemeinsam ist dabei, dass sie durch Polyaddition von Polyaminen oder Polyolen an mehrwertige Isocyanate hergestellt werden. Durch geschickte Auswahl der Polyamin- bzw. Polyolkomponente kann dabei das Eigenschaftsprofil des erhaltenen Polyurethans gezielt gesteuert werden.

Als nachteilig erweist sich die hohe Reaktivität der mehrwertigen Isocyanate, die zu einer hohen Feuchtigkeitsempfindlichkeit führt, die man sich für die Herstellung von Schäumen zunutze macht, welche für andere Anwendungen, wie z. B. Coatings, jedoch nicht erwünscht ist. Zwar sind mehrwertige Isocyanate unter wasserfreien Bedingungen über längere Zeit lagerbar, jedoch tritt die Reaktion mit Wasser bei der Aushärtung ein, so dass sehr trockenes Arbeiten notwendig ist. Über die Feuchtigkeitsempfindlichkeit hinaus neigen insbesondere die aromatischen Isocyanate (MDI, TDI) zu Verfärbungen. Problematisch ist auch die gesundheitliche Bedenklichkeit einiger Diisocyanate. So ist bekannt, dass Diisocyanate bei Hautkontakt oder Inhalation Allergien auslösen können. Aus diesem Grund wurden Oligomere von Diisocyanaten entwickelt, die aufgrund ihrer geringeren Flüchtigkeit leichter zu handhaben sind. Dennoch besteht grundsätzlich ein Bedarf an Alternativen für die aus dem Stand der Technik bekannten Polyisocyanate.

Alkyliden-1,3-dioxolan-2-one, die im Folgenden auch als exo-Vinylencarbonate bezeichnet werden, wurden verschiedentlich in der Literatur beschrieben, beispielsweise in DE 1098953, DE 3433403, EP 837062, JP 2006137733, JP 2008222619, J. Org. Chem. 2007, 72, 647-649, Angew. Chem. 2009, 121, 4258-4261, Eur. J. Org. Chem. 2007, 2604-2607, Eur. J. Org. Chem. 2008, 2309-2312, Org. Lett. 2006, 8, 515-518. Alkyliden-1,3-dioxolan-2-one werden dort als Synthesebausteine für die Herstellung von Wirk- und Effektstoffen vorgeschlagen.

Die WO 2011/157671 beschreibt die Verwendung von Alkyliden-1,3-dioxolan-2-onen zusammen mit aminischen Härtern als Additive in Epoxidharzzusammensetzungen.

Die WO 96/26224 beschreibt die Copolymerisation von 4-Vinyl-1,3-dioxolan-2-onen mit ethylenisch ungesättigten Comonomeren. Die dabei erhaltenen Polymere weisen 1,3-Dioxolan-2-on-Gruppen auf und werden zusammen mit aminofunktionellen Vernetzern zur Herstellung von Beschichtungen eingesetzt.

Aus der US 2003/100687 sind 4-(Meth)acryloxyalkyl-1,3-dioxolan-2-one bekannt, die mit ethylenisch ungesättigten Comonomeren zu Copolymeren polymerisiert werden, welche über Alkyloxycarbonyl-Einheiten gebundene 1,3-Dioxolan-2-on-Gruppen aufweisen. Die Polymere werden mit aminischen Verbindungen umgesetzt, wobei man Pfropfpolymere erhält, die Urethan- und Hydroxylgruppen aufweisen. Die Pfropfpolymere werden in Beschichtungsmitteln eingesetzt.

Die Reaktivität der aus dem Stand der Technik bekannten Polymere mit 1,3-Dioxolan-2-on-Gruppen ist jedoch nicht zufriedenstellend, insbesondere bei der Reaktion mit Aminen. Zudem werden bei der Umsetzung von 1,3-Dioxolan-2-onen mit beispielsweise Aminen oder Alkoholen Hydroxylgruppen gebildet, die sich in verschiedenen Anwendungen als nachteilig erweisen können.

Es wurde nun überraschenderweise gefunden, dass die im Folgenden näher beschriebenen Verbindungen der Formel I, die eine Alkyliden-1,3-dioxolan-2-on-Gruppe und eine weitere ethylenisch ungesättigte Doppelbindung aufweisen, unter Erhalt der Alkyliden-1,3-dioxolan-2-on-Gruppe radikalisch polymerisiert werden können. Dies ist überraschend, da in der Literatur verschiedentlich beschrieben wird, dass die Methylengruppe in Methylen-1,3-dioxolan-2-onen unter radikalischen Bedingungen polymerisiert, siehe beispielsweise in Journal of Network Polymer, Japan 2005, 26, 132-137, Makromol. Chem., Rapid Commun. 1989, 10, 453-456.

Dementsprechend betrifft ein erster Aspekt der Erfindung die nachstehend definierten Verbindungen der allgemeinen Formel I, 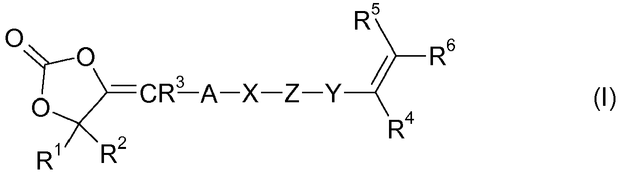 worin
- R¹ und R²: unabhängig voneinander für Wasserstoff, C₁-C₆-Alkyl, C₁-C₄-Alkoxy-C₁-C₄-alkyl, C₅-C₆-Cycloalkyl, Phenyl oder Phenyl-C₁-C₄-alkyl stehen;
- R³: für Wasserstoff, C₁-C₆-Alkyl, C₁-C₄-Alkoxy-C₁-C₄-alkyl, C₅-C₆-Cycloalkyl, Phenyl oder Phenyl-C₁-C₄-alkyl steht, wobei R³ insbesondere für Wasserstoff steht;
- R⁴: für Wasserstoff, C₁-C₄-Alkyl, CH₂COOR⁸, Phenyl oder Phenyl-C₁-C₄-alkyl steht;
- R⁵ und R⁶: unabhängig voneinander für Wasserstoff oder C₁-C₄-Alkyl stehen oder einer der Reste R⁵ oder R⁶ auch für COOR⁸ oder CH₂COOR⁸ stehen kann;
- A: für eine chemische Bindung oder C₁-C₄-Alkandiyl steht, wobei A insbesondere für C₁-C₄-Alkandiyl steht;
- X: für O oder NR⁷ steht;
- Z: für eine chemische Bindung, PO₂, SO₂ oder C=O steht, wobei Z insbesondere für C=O steht;
- Y: für eine chemische Bindung, CH₂ oder CHCH₃ steht, wobei Y insbesondere für eine chemische Bindung steht;
- R⁷: sofern vorhanden, für C₁-C₆-Alkyl steht;
- R⁸: sofern vorhanden, für Wasserstoff oder C₁-C₆-Alkyl steht.

Die bei der Homo- oder Copolymerisation der Verbindungen der Formel I erhaltenen Homo- oder Copolymere weisen in der Regel mehrere funktionelle Gruppen der Formel I', die an das aus Kohlenstoff-Atomen aufgebaute Polymerrückgrat angebunden sind, auf.

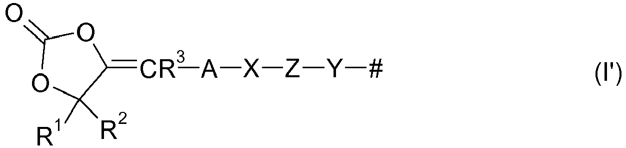

In Formel I' steht # für die Anbindung an das Polymerrückgrat und R¹, R², R³, A, X, Z und Y weisen die hier und im Folgenden genannten Bedeutungen auf. Derartige Polymere zeigen gegenüber Verbindungen, die funktionelle Gruppen F aus der Gruppe der aliphatischen Hydroxylgruppen, primären und sekundären Aminogruppen, Phosphingruppen, Phosphonatgruppen und Mercaptangruppen aufweisen, eine hohe Reaktivität auf, ohne die mit Isocyanaten verbundenen Nachteile zu besitzen. Sie eignen sich daher in besonderer Weise als Ersatz für polyfunktionelle Isocycanate in zahlreichen Anwendungen, insbesondere für 2K-Bindemittel. Sie sind daher ebenfalls Gegenstand der vorliegenden Erfindung.

Hier und im Folgenden gibt das zur Definition von Substituenten und chemischen Verbindungen verwendete Präfix "Cₙ-Cₘ-" die Anzahl möglicher C-Atome des Substituenten bzw. der Verbindung an.

Werden keine anderen Angaben gemacht, so gelten im Rahmen der vorliegenden Erfindung für die im Zusammenhang mit den Substituenten verwendeten Begriffe die folgenden allgemeinen Definitionen:

"Alkyl" steht für einen linearen oder verzweigten Alkylrest mit beispielsweise 1 bis 4 (C₁-C₄-Alkyl), 1 bis 6 (C₁-C₆-Alkyl) oder 1 bis 20 Kohlenstoffatomen (C₁-C₂₀-Alkyl). Beispiele für C₁-C₄-Alkyl sind Methyl, Ethyl, n-Propyl, iso-Propyl, n-Butyl, 2-Butyl, isoButyl, tert.-Butyl (2-Methylpropan-2-yl). Beispiele für C₁-C₆-Alkyl sind neben den für C₁-C₄-Alkyl genannten Bedeutungen weiterhin n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl. Beispiele für C₁-C₂₀-Alkyl sind neben den für C₁-C₆-Alkyl genannten Bedeutungen weiterhin Heptyl, Octyl, 2-Ethylhexyl, Nonyl, Decyl, Undecyl, Dodecyl, Tridecyl, Tetradecyl, Pentadecyl, Hexadecyl, Heptadecyl, Octadecyl, Nonadecyl, Eicosyl und deren Konstitutionsisomere.

"C₁-C₄-Alkoxy-C₁-C₄-alkyl" steht für eine über ein Sauerstoffatom gebundene Alkylgruppe mit 1 bis 4 Kohlenstoffatomen, wie z. B. Methoxy, Ethoxy, n-Propoxy, 1-Methylethoxy (Isopropoxy), n-Butoxy, 1-Methylpropoxy (sec.-Butoxy), 2-Methylpropoxy (Isobutoxy) oder 1,1-Dimethylethoxy (tert.-Butoxy), die in Form einer Etherbindung über den Sauerstoff an eine wie zuvor definierte C₁-C₄-Alkylgruppe gebunden ist. Beispiele sind Methoxymethyl, 2-Methoxyethyl, Ethoxymethyl, 3-Methoxypropyl, 3-Ethoxypropyl.

"C₅-C₆-Cycloalkyl" steht für einen cyclischen Alkylrest mit 5 bis 6 Kohlenstoffatomen. Beispiele sind Cyclopentyl und Cyclohexyl.

"Phenyl-C₁-C₄-alkyl" steht für eine Phenylgruppe, die an eine wie zuvor definierte C₁-C₄-Alkylgruppe gebunden ist. Beispiele sind Benzyl, Phenylethyl, Phenylpropyl, Phenylbutyl.

"C₁-C₄-Alkandiyl" steht für ein Alkandiyl mit 1 bis 4 Kohlenstoffatomen. Beispiele sind Methandiyl, 1,1-Ethandiyl, 1,2-Ethandiyl, 1-Methyl-1,1-ethandiyl, 1-Methyl-1,2-ethandiyl, 1,3-Propandiyl, 1,4-Butandiyl, 1,1-Dimethyl-1,2-ethandiyl und 1,2-Dimethyl-1,2-ethandiyl.

"C₁-C₈-Alkoxy" steht für eine über ein Sauerstoffatom gebundene Alkylgruppe mit 1 bis 8 Kohlenstoffatomen. Beispiele sind Methoxy, Ethoxy, n-Propoxy, 1-Methylethoxy (Isopropoxy), n-Butoxy, 1-Methylpropoxy (sec.-Butoxy), 2-Methylpropoxy (Isobutoxy), 1,1-Dimethylethoxy (tert.-Butoxy), n-Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, 2-Ethylpropoxy, n-Hexoxy, 1-Methylpentoxy, 2-Methylpentoxy, 3-Methylpentoxy, 4-Methylpentoxy, 1-Ethylbutoxy, 2-Ethylbutoxy, 3-Ethylbutoxy, 1,2-Dimethylbutoxy, 1,3-Dimethylbutoxy, 2,3-Dimethylbutoxy, 1-Ethyl-2-methylpropoxy und 1-Isopropylpropoxy.

"C₁-C₄-Alkylcarbonyl" steht für einen über eine Carbonylgruppe gebundenen C₁-C₄-Alkylrest wie zuvor definiert, z. B. für Acetyl, Propionyl, Butyryl, Pivaloyl etc.

Im Hinblick auf bevorzugte Ausführungsformen der Erfindung weisen die Reste bzw. Gruppen R¹, R², R³, R⁴, R⁵, R⁶, R⁷, R⁸, A, X, Z und Y in den Verbindungen der Formel I und den Gruppen der Formel I' unabhängig voneinander vorzugsweise eine oder mehrere oder alle der folgenden Bedeutungen auf:
- R¹: steht für Wasserstoff oder C₁-C₆-Alkyl, insbesondere für Wasserstoff oder C₁-C₄-Alkyl und speziell für Methyl oder Ethyl;
- R²: steht für Wasserstoff oder C₁-C₆-Alkyl, insbesondere für C₁-C₄-Alkyl und speziell für Methyl oder Ethyl;
- R³: steht für Wasserstoff;
- A: steht für C₁-C₄-Alkandiyl, insbesondere für Methandiyl, 1,2-Ethandiyl oder 1,3-Propandiyl;
- X: steht für O;
- Z: steht für C=O;
- Y: steht für eine chemische Bindung;
- R⁴: steht für Wasserstoff oder C₁-C₄-Alkyl, insbesondere für Wasserstoff oder Methyl;
- R⁵: steht für Wasserstoff;
- R⁶: steht für Wasserstoff;
- R⁷: sofern vorhanden, steht für C₁-C₄-Alkyl;
- R⁸: sofern vorhanden, steht für C₁-C₄-Alkyl.

Die Herstellung der Verbindungen der Formel I gelingt in der Regel durch das im Folgenden näher erläuterte Verfahren, das ebenfalls Gegenstand der vorliegenden Erfindung ist. Bei diesem Verfahren setzt man eine Verbindung der allgemeinen Formel II mit einer Verbindung der allgemeinen Formel III um:

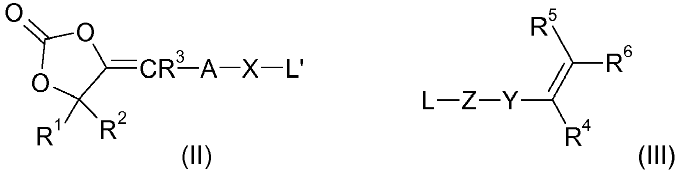

In Formel II steht L' für Wasserstoff oder eine Hydroxyl- oder Amino-Schutzgruppe, z. B. eine C₁-C₄-Alkylcarbonylgruppe. Die Variablen A, X, R¹, R² und R³ weisen die oben genannten Bedeutungen, insbesondere die als bevorzugt genannten Bedeutungen, auf.

In Formel III steht L für eine nucleophil verdrängbare Abgangsgruppe, beispielsweise Halogen, OH oder C₁-C₈-Alkoxy. Die Variablen Y, Z, R⁴, R⁵ und R⁶ weisen die oben genannten Bedeutungen, insbesondere die als bevorzugt genannten Bedeutungen, auf.

Die Umsetzung der Verbindungen der Formeln II und III kann in Analogie zu bekannten Verfahren der nucleophilen Substitution durchgeführt werden. Sofern L' für eine Hydroxyl- oder Amino-Schutzgruppe steht, wird in der Regel diese Schutzgruppe vor der Umsetzung von Verbindung II mit Verbindung III entfernt, oder es werden Reaktionsbedingungen gewählt, unter denen die Schutzgruppe abgespalten wird, so dass der eigentliche Reaktant die Verbindung der Formel II ist, worin L' für Wasserstoff steht.

Gemäß einer bevorzugten Ausführungsform der Erfindung stehen in Formel III die Variablen Z für C=O und L für OH oder C₁-C₈-Alkoxy. In diesem Fall gelingt die Umsetzung von Verbindung III mit Verbindung II, gegebenenfalls nach Entfernung der Hydroxyl- oder Amino-Schutzgruppe, im Sinne einer Amidierung oder Veresterungs- bzw. Umesterungsreaktion.

Insbesondere eignet sich die Veresterung bzw. Umesterung für die Herstellung von Verbindungen der Formel I, worin Z für C=O und X für O steht, A für C₁-C₄-Alkandiyl steht, R⁴ Wasserstoff oder C₁-C₄-Alkyl, speziell Wasserstoff oder Methyl, bedeutet und R⁵ und R⁶ für Wasserstoff stehen. In diesem Fall sind bevorzugte Reaktanten der Formel II ausgewählt unter den C₁-C₈-Alkylestern der Acrylsäure und der Methacrylsäure, im Folgenden (Meth)acrylsäure-C₁-C₈-alkylester, z. B. (Meth)acrylsäuremethyl-, -ethyl-, -n-butyl- und -2-ethylhexylester und ganz besonders bevorzugt (Meth)acrylsäure-C₁-C₄-alkylester, z. B. (Meth)acrylsäuremethyl-, -ethyl- und -n-butylester.

Gemäß einer besonders bevorzugten Ausführungsform der Erfindung stehen in Formel I die Variablen L für OH oder C₁-C₈-Alkoxy, Z für C=O und in Formel I X für O und die Umsetzung von Verbindung II mit Verbindung III wird unter den Bedingungen einer Veresterung oder Umesterung durchgeführt. In einer speziellen Ausgestaltung dieser Ausführungsform steht L' in Formel II für Wasserstoff oder eine C₁-C₄-Alkylcarbonylgruppe, speziell eine Acetylgruppe.

In einer bevorzugten Ausführungsform des erfindungsgemäßen Verfahrens erfolgt die Herstellung der Verbindungen der Formel I durch Veresterung bzw. Umesterung unter Enzym-Katalyse.

Die Enzym-katalysierte Veresterung bzw. Umesterung kann in Analogie zu den in Biotechnol. Lett. 1990, 12, 825-830, Biotechnol. Lett. 1994, 16, 241-246, US 5240835, WO 2004/05088 oder DE 102009003035 beschriebenen Methoden durchgeführt werden, auf die hier vollumfänglich Bezug genommen wird.

Für die Enzym-katalysierte Veresterung bzw. Umesterung einsetzbare Enzyme (E) sind beispielsweise ausgewählt unter Hydrolasen, Esterasen (E.C. 3.1.-.-), Lipasen (E.C. 3.1.1.3), Glykosylasen (E.C. 3.2.-.-) und Proteasen (E.C. 3.4.-.-) in freier oder auf einem Träger chemisch oder physikalisch immobilisierter Form, bevorzugt Lipasen, Esterasen oder Proteasen. Besonders bevorzugt sind Novozym^{®} 435 der Fa. Novozymes (Lipase aus Candida antartica B) oder Lipase aus Aspergillus sp., Aspergillus niger sp., Mucor sp., Penicilium cyclopium sp., Geotricum candidum sp., Rhizopus javanicus, Burholderia sp., Candida sp., Pseudomonas sp. oder Schweinepankreas, ganz besonders bevorzugt sind Lipase aus Candida antartica B oder aus Burholderia sp.

Der Enzymgehalt im Reaktionsmedium liegt in der Regel im Bereich von etwa 0,1 bis 10 Gew.-%, bezogen auf die Summe der eingesetzten Reaktanten der Formel II und III.

Die Herstellung der Verbindungen der Formel I kann auch durch konventionelle Veresterung oder Umesterung unter den hierfür üblichen Reaktionsbedingungen einer säurekatalysierten Veresterung oder einer säure- oder basenkatalysierten Umesterung erfolgen.

Als saure Katalysatoren für eine säurekatalysierte Veresterung eignen sich vor allem Protonensäuren, beispielsweise Schwefelsäure, Natriumhydrogensulfat, Salzsäure, Phosphorsäure, Mononatriumdihydrogenphosphat, Dinatriumhydrogenphosphat, Pyrophosphorsäure, phosphorige Säure, unterphosphorige Säure, Methansulfonsäure, Trifluormethansulfonsäure, p-Toluolsulfonsäure sowie deren Gemische. Geeignet sind auch Lewissäuren wie z. B. Ti- und Sn-Verbindungen. Außerdem geeignet sind saure lonentauscherharze, z. B. sulfonierte oder carboxylierte lonentauscherharze, jeweils in ihrer sauren Form.

Als basische Katalysatoren für eine Umesterung eignen sich Metallhydroxide und/oder -alkoholate, insbesondere von Metallen der 1., 2. und 13. Gruppe des Periodensystems, beispielsweise Alkalimetallhydroxide wie NaOH oder KOH sowie Alkalimetall- und Erdalkalimetallalkanolate, insbesondere die entsprechenden Methanolate oder Ethanolate wie Natrium- oder Kaliummethanolat oder Natrium- oder Kaliumethanolat. Außerdem geeignet sind ionenaustauschende Harze.

Die sauren oder basischen Katalysatoren werden in der Regel in einer Konzentration von 0,0001 Gew.-% bis 20 Gew.-%, bevorzugt 0,001 Gew.-% bis 10 Gew.-%, bezogen auf die gesamte Reaktionsmischung, eingesetzt.

Die Veresterungs- bzw. Umesterungsreaktion von II mit III kann beispielsweise als Batch-Verfahren gestaltet werden. Hierbei wird man in der Regel die Verbindungen der Formeln II und III in ein Reaktionsgefäß geben und unter Zugabe des Katalysators bzw. des Enzyms miteinander umsetzten. Alternativ kann die Veresterungs- bzw. Umesterungsreaktion als Semi-Batch-Verfahren gestaltet werden. Hierzu kann man beispielsweise einen der Reaktanten, z. B. die Verbindung II oder Verbindung III, sowie den Katalysator bzw. das Enzym vorlegen und den anderen Reaktanten im Laufe der Reaktion zuführen. Außerdem kann die Verbindung der Formel I durch kontinuierliche Umsetzung der Verbindung II mit der Verbindung III hergestellt werden. Hierzu wird man beispielsweise die Verbindungen II und III kontinuierlich einer Reaktionszone, welche den Katalysator enthält, zuführen und die Verbindung der Formel I, gegebenenfalls zusammen mit den bei der Reaktion gebildeten Koppelprodukten, z. B. Alkohol oder Ester, kontinuierlich der Reaktionszone entnehmen. Gegebenenfalls wird man den Katalysator bzw. das Enzym ebenfalls der Reaktionszone zuführen. Sowohl bei der Semi-Batch-, als auch bei der kontinuierlichen Umsetzung kann man die Reaktanten, d. h. die Verbindungen der Formeln II und III, vorzugsweise in flüssiger Phase, durch eine Reaktionszone führen, welche den Katalysator bzw. das Enzym als stationäre Phase enthält.

Die Reaktionszeit hängt unter anderem von der Temperatur, der verwendeten Menge und der Aktivität des Säure-, Basen- bzw. Enzymkatalysators und vom geforderten Umsatz ab sowie von der Struktur der Verbindung II. Bevorzugt wird die Reaktionszeit so angepasst, dass der Umsatz der Verbindung II mindestens 70 %, bevorzugt mindestens 80 %, besonders bevorzugt mindestens 90 %, ganz besonders bevorzugt mindestens 95 % und insbesondere mindestens 97 % beträgt. In der Regel sind dafür 1 bis 48 Stunden, bevorzugt 1 bis 12 Stunden und besonders bevorzugt 1 bis 6 Stunden ausreichend.

Die Enzym-katalysierte oder konventionell katalysierte Veresterung bzw. Umesterung erfolgt im Allgemeinen bei Temperaturen im Bereich von 0 bis 100 °C, bevorzugt 20 bis 80 °C und besonders bevorzugt 20 bis 70 °C.

Das molare Verhältnis von Verbindung II zu Verbindung III kann in einem weiten Bereich variiert werden. Vorzugsweise wird die Verbindung III im Überschuss bezogen auf die Stöchiometrie der Umsetzung eingesetzt. Im Allgemeinen liegt das molare Verhältnis von Verbindung II zu Verbindung III im Bereich von 1 : 100 bis 1 : 1, bevorzugt 1 : 50 bis 1 : 1, besonders bevorzugt 1 : 20 bis 1 : 1. Bevorzugt liegt die Verbindung der Formel III im Überschuss vor, so dass sie zusammen mit dem freiwerdenden Koppelprodukt, in der Regel ein Alkohol oder das bei einer Umesterung gebildete Ester-Koppelprodukt (wenn X-L' in Formel II für Alkylcarbonyloxy und Y-L in Formel III für Alkoxycarbonyl steht), unter vermindertem Druck, beispielsweise als Azeotrop, abdestilliert werden kann. Zusätzlich oder alternativ kann das freiwerdende Wasser bzw. der Alkohol bzw. der Ester z. B. mittels Molekularsiebs gebunden werden. Auf diese Weise wird das Reaktionsgleichgewicht zugunsten der Verbindung der Formel I verschoben.

Die Enzym-katalysierte sowie die konventionell katalysierte Veresterung bzw. Umesterung können in organischen Lösungsmitteln oder deren Gemischen oder ohne Zusatz von Lösungsmitteln durchgeführt werden. Die Ansätze sind in der Regel weitgehend wasserfrei (d. h. unter 10 Vol.-%, bevorzugt unter 5 Vol.-%, besonders bevorzugt unter 1 Vol.-% Wassergehalt).

Der Anteil organischer Lösungsmittel am Reaktionsgemisch kann beispielsweise 0,1 bis 50 Gew.-% betragen und liegt, sofern ein Lösungsmittel eingesetzt wird, bevorzugt im Bereich von 0,5 bis 30 Gew.-% oder im Bereich von 1 bis 10 Gew.-%. Vorzugsweise wird kein oder weniger als 1 Gew.-% organisches Lösungsmittel der Enzym- oder konventionell katalysierten Veresterung bzw. Umesterung zugesetzt.

Die Herstellung der Verbindung I kann in Gegenwart mindestens eines Polymerisationsinhibitors durchgeführt werden. Als Polymerisationsinhibitoren können beispielsweise 4-Methoxyphenol (MeHQ), Hydrochinon, 2,5-Di-tert.-butylhydrochinon, 2,6-Di-tert.-butyl-p-cresol, Nitrosoverbindungen wie Isoacrylnitrat, Nitrosodiphenylamin, N-Nitrosocyclohexylhydroxylamin, Methylenblau, Phenothiazin oder Diphenylamin eingesetzt werden. Bevorzugt wird 4-Methoxyphenol (MeHQ) als Polymerisationsinhibitor eingesetzt.

Die Polymerisationsinhibitoren werden im Allgemeinen, bezogen auf die Menge der Verbindungen der Formel III, von 1 bis 10000 ppm, bevorzugt von 10 bis 5000 ppm, besonders bevorzugt von 30 bis 2500 ppm und insbesondere von 50 bis 1500 ppm eingesetzt.

Die Verbindungen der Formel III sind bekannt und in der Regel kommerziell erhältlich.

Die Herstellung der Verbindungen der Formel II kann in Analogie zu bekannten Verfahren zur Herstellung von Alkyliden-1,3-dioxolan-2-onen erfolgen, wie sie beispielsweise im eingangs zitierten Stand der Technik beschrieben werden. Bevorzugte Verbindungen der Formel II, worin R³ für Wasserstoff steht, können beispielsweise durch Umsetzung der Verbindung der Formel IV mit CO₂, vorzugsweise unter Einsatz eines Katalysators, hergestellt werden (siehe Schema 1):

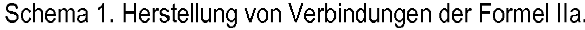

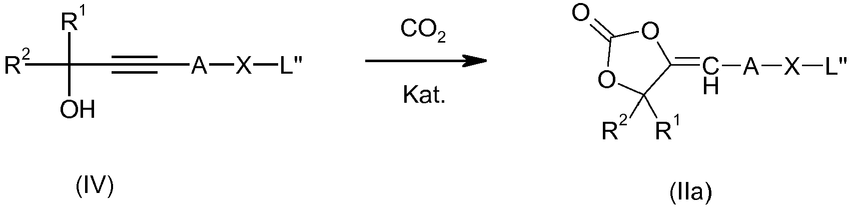

In Schema 1 haben R¹, R², A und X die zuvor genannten Bedeutungen. L" steht für eine Alkohol- oder Amino-Schutzgruppe und insbesondere für C₁-C₄-Alkylcarbonyl, speziell für Acetyl. X steht insbesondere für Sauerstoff. A steht insbesondere für C₁-C₄-Alkandiyl.

Als Katalysatoren kommen grundsätzlich Übergangsmetallkatalysatoren in Frage, die als aktives Metall beispielsweise Silber, Kupfer, Gold, Palladium oder Platin enthalten, z. B. Silbersalze wie Silberacetat, Silbercarbonat, Kupfer(II)-Salze wie Kupferacetat oder Kupfer(I)-Halogenide wie Cul, CuBr, CuCl, weiterhin Palladium(0)-Katalysatoren, wobei die vorgenannten Übergangsmetall-Verbindungen gegebenenfalls in Kombination mit einem organischen Amin, z. B. einem Tri-C₁-C₆-alkylamin wie Triethylamin oder einer Amidin-Base wie 1,5-Diazabicyclo[4.3.0]non-5-en (DBN) oder 1,8-Diazabicyclo[5.4.0]undec-7-en (DBU), oder mit einem organischen Phosphin, z. B. Trialkylphosphinen oder Triarylphosphinen wie Tributylphosphin und Triphenylphosphin, oder in Kombination mit einer Mischung aus einem der vorgenannten Phosphine mit einem Ammoniumsalz, wie beispielsweise Tri-C₁-C₆-alkylammoniumhalogeniden oder Tetra-C₁-C₆-alkylammoniumhalogeniden, eingesetzt werden können. Als Katalysatoren kommen weiterhin organische Phosphine als solche, z. B. Trialkylphosphine oder Triarylphosphine wie Tributylphosphin oder Triphenylphosphin, sowie sterisch gehinderte Carbene, z. B. 1,3-substituierte 2,3-Dihydroimidazol-2-yliden-Verbindungen wie 1,3-Diisopropyl-2,3-dihydro-4,5-imidazol-2-yliden oder deren CO₂-Addukte, sowie Kombinationen davon mit den vorgenannten Phosphinen in Betracht. Die Reaktion kann drucklos oder vorzugsweise unter erhöhtem Druck, z. B. bei 50 bis 500 bar, oder in überkritischem CO₂ durchgeführt werden. Bezüglich der Reaktionsbedingungen wird auf die zuvor genannte Literatur verwiesen.

Anstelle von CO₂ kann auch ein Carbonsäureanhydrid wie beispielsweise Bis-(tert.-butyl)dikohlensäureanhydrid (Boc₂O) eingesetzt werden. In diesem Fall erfolgt die Umsetzung üblicherweise in zwei Stufen, wobei man in der ersten Stufe die Verbindung IV mit einem Ester des Biskohlensäureanhydrids, z. B. mit Boc₂O, in Gegenwart einer Base, beispielsweise Natriumhydrid, umsetzt und den dabei erhaltenen Ester in Gegenwart eines Übergangsmetallkatalysators, z. B. eines goldhaltigen Katalysators, cyclisiert. Eine derartige Vorgehensweise ist beispielsweise in Org. Lett. 2006, 8, 515-518 beschrieben, auf die hiermit Bezug genommen wird.

Gegenstand der Erfindung sind auch Polymere, welche wenigstens eine Verbindung der Formel I in einpolymerisierter Form enthalten. Derartige Polymere sind typischerweise durch Homo- oder Copolymerisation ethylenisch ungesättigter Monomere M erhältlich, wobei die ethylenisch ungesättigten Monomere M wenigstens ein Monomer der allgemeinen Formel I (Monomer a) und gegebenenfalls ein oder mehrere ethylenisch ungesättigte Comonomere umfassen. Derartige Polymere weisen ein aus Kohlenstoff-Atomen aufgebautes Polymerrückgrat auf, an das in der Regel wenigstens zwei Gruppen der allgemeinen Formel I', z. B. 2 bis 1000 Gruppen der Formel I', gebunden sind. Dementsprechend weist ein solches Polymer wenigstens zwei Wiederholungseinheiten der Formel I" auf:

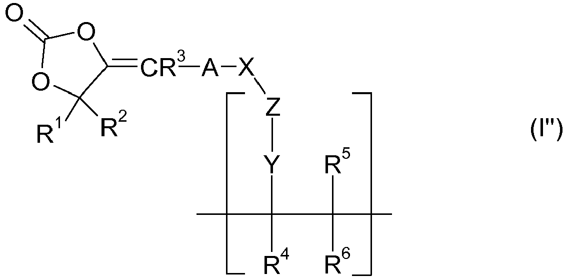

Der Anteil der Verbindungen der allgemeinen Formel I beträgt vorzugsweise wenigstens 10 Gew.-%, insbesondere wenigstens 15 Gew.-% und speziell wenigstens 20 Gew.-%, bezogen auf die Gesamtmenge der zu polymerisierenden Monomere M, und kann bis zu 100 Gew.-% betragen. Dementsprechend beträgt der Anteil der Wiederholungseinheiten der allgemeinen Formel I" vorzugsweise wenigstens 10 Gew.-%, insbesondere wenigstens 15 Gew.-% und speziell wenigstens 20 Gew.-%, bezogen auf die Gesamtmenge der im Polymer enthaltenen Wiederholungseinheiten, und kann bis zu 100 Gew.-% betragen.

Eine erste Ausführungsform der Erfindung betrifft Homopolymere einer Verbindung der Formel I, d. h., die Polymere sind, abgesehen von ihren Endgruppen, ausschließlich aus einer bestimmten Wiederholungseinheit der allgemeinen Formel I" aufgebaut.

Eine zweite Ausführungsform der Erfindung betrifft Copolymere wenigstens zwei voneinander verschiedener Verbindungen der Formel I, d. h., die Polymere sind, abgesehen von ihren Endgruppen, ausschließlich aus zwei oder mehreren voneinander verschiedenen Wiederholungseinheiten der allgemeinen Formel I" aufgebaut.

Eine dritte Ausführungsform der Erfindung betrifft Copolymere, die aus wenigstens einer Verbindungen der Formel I mit wenigstens einem, z. B. 1, 2 oder 3, davon verschiedenen ethylenisch ungesättigten Comonomer, d. h., Verbindungen, die keine Gruppe der Formel I' aufweisen, aufgebaut sind. Derartige erfindungsgemäße Copolymere weisen neben Wiederholungseinheiten der Formel I" auch Wiederholungseinheiten auf, die aus dem polymerisierten Comonomer abgeleitet sind.

Geeignete Comonomere sind vor allem monoethylenisch ungesättigte Comonomere, aber auch konjugiert diethylenisch ungesättigte Verbindungen. Diese werden im Folgenden auch als Comonomere b bezeichnet. Zu den Comonomeren b zählen beispielsweise:
b1 monoethylenisch ungesättigte C₃-C₈-Mono- und C₄-C₈-Dicarbonsäuren wie beispielsweise Acrylsäure, Methacrylsäure, Vinylessigsäure, Crotonsäure, Fumarsäure, Maleinsäure und Itaconsäure;
b2 Amide monoethylenisch ungesättigter C₃-C₈-Mono- und C₄-C₈-Dicarbonsäuren wie Acrylamid, Methacrylamid, Fumarsäureamid und Maleinimid;
b3 Anhydride monoethylenisch ungesättigter C₄-C₈-Dicarbonsäuren wie Maleinsäureanhydrid;
b4 Hydroxy-C₂-C₄-alkylester monoethylenisch ungesättigter C₃-C₈-Mono- und C₄-C₈-Dicarbonsäuren wie 2-Hydroxyethylacrylat, 2-Hydroxypropylacrylat, 3-Hydroxypropylacrylat, 2-Hydroxybutylacrylat, 4-Hydroxybutylacrylat, 2-Hydroxyethylmethacrylat, 2-Hydroxypropylmethacrylat, 3-Hydroxypropylmethacrylat, 2-Hydroxybutylmethacrylat, 4-Hydroxybutylmethacrylat;
b5 monoethylenisch ungesättigte Sulfonsäuren und deren Salze, z. B. Vinylsulfonsäure, Allylsulfonsäure, Methallylsulfonsäure, Styrolsulfonsäure, 2-Acrylamido-2-methylpropansulfonsäure, 2-Methacrylamido-2-methylpropansulfonsäure, 2-Acrylamidoethansulfonsäure, 2-Methacrylamidoethansulfonsäure, 2-Acryloxyethansulfonsäure, 2-Methacryloxyethansulfonsäure, 3-Acryloxypropansulfonsäure und 2-Methacryloxypropansulfonsäure;
b6 monoethylenisch ungesättigte Nitrile mit 3 bis 5 C-Atomen wie Acrylnitril und Methacrylnitril;
b7 N-Vinylheterocyclen wie N-Vinylpyrrolidon, N-Vinylcaprolactam, N-Vinylimidazol;
b8 monoethylenisch ungesättigte Verbindungen mit wenigstens einer Poly-C₂-C₄-alkylenoxid-Gruppe, beispielsweise Vinyl- und Allylether von Poly-C₂-C₄-alkylenglykolen oder C₁-C₁₀-Alkyl-poly-C₂-C₄-alkylenglykolen, Ester monoethylenisch ungesättigter Mono- und Dicarbonsäuren mit 3 bis 8 C-Atomen mit Poly-C₂-C₄-alkylenglykolen oder C₁-C₁₀-Alkyl-poly-C₂-C₄-alkylenglykolen;
b9 vinylaromatische Kohlenwasserstoffe wie Styrol, α-Methylstyrol und die Vinyltoluolisomere;
b10 Ester monoethylenisch ungesättigter C₃-C₈-Monocarbonsäuren mit C₁-C₂₀-Alkanolen, C₅-C₈-Cycloalkanolen, Phenyl-C₁-C₄-alkanolen oder Phenoxy-C₁-C₄-alkanolen, beispielsweise Ester der Acrylsäure mit C₁-C₂₀-Alkanolen wie Methylacrylat, Ethylacrylat, n-Butylacrylat, 2-Butylacrylat, Isobutylacrylat, tert.-Butylacrylat, 2-Ethylhexylacrylat, Decylacrylat, Laurylacrylat und Stearylacrylat, Ester der Acrylsäure mit C₅-C₁₀-Cycloalkanolen wie Cyclohexylacrylat, Ester der Acrylsäure mit Phenyl-C₁-C₄-alkanolen wie Benzylacrylat, 2-Phenylethylacrylat und 1-Phenylethylacrylat, Ester der Acrylsäure mit Phenoxy-C₁-C₄-alkanolen wie 2-Phenoxyethylacrylat, Ester der Methacrylsäure mit C₁-C₂₀-Alkanolen, vorzugsweise C₁-C₁₀-Alkanolen, wie Methylmethacrylat, Ethylmethacrylat, n-Butylmethacrylat, 2-Butylmethacrylat, Isobutylmethacrylat, tert.-Butylmethacrylat, 2-Ethylhexylmethacrylat, Decylmethacrylat, Laurylmethacrylat und Stearylmethacrylat, Ester der Methacrylsäure mit C₅-C₁₀-Cycloalkanolen wie Cyclohexylmethacrylat, Ester der Methacrylsäure mit Phenyl-C₁-C₄-alkanolen wie Benzylmethacrylat, 2-Phenylethylmethacrylat und 1-Phenylethylmethacrylat und Ester der Methacrylsäure mit Phenoxy-C₁-C₄-alkanolen wie 2-Phenoxyethylmethacrylat;
b11 Diester monoethylenisch ungesättigter C₄-C₈-Dicarbonsäuren mit C₁-C₂₀-Alkanolen, C₅-C₈-Cycloalkanolen, Phenyl-C₁-C₄-alkanolen oder Phenoxy-C₁-C₄-alkanolen;
b12 C₁-C₂₀-Alkylamide und Di-C₁-C₂₀-alkylamide monoethylenisch ungesättigter C₃-C₈-Monocarbonsäuren, insbesondere die C₁-C₂₀-Alkylamide und Di-C₁-C₂₀-alkylamide der Acrylsäure und der Methacrylsäure, z. B. Ethylacrylamid, Dimethylacrylamid, Diethylacrylamid, n-Propylacrylamid, n-Butylacrylamid, Laurylacrylamid, Stearylacrylamid, Ethylmethacrylamid, Dimethylmethacrylamid, Diethylmethacrylamid, n-Propylmethacrylamid, n-Butylmethacrylamid, Laurylmethacrylamid, Stearylmethacrylamid;
b13 Vinylester aliphatischer Carbonsäuren mit 1 bis 20 C-Atomen, z. B. Vinylacetat, Vinylpropionat, Vinylbutyrat, Vinylhexanoat, Vinyllaurat und Vinylstearat;
b14 konjugiert diethylenisch ungesättigte C₄-C₁₀-Olefine wie Butadien und Isopren;
b15 C₂-C₂₀-Olefine wie Ethylen, Propen, 1-Buten, 2-Buten, Isobuten, 1-Hexen, 1-Octen, Diisobuten und 1-Decen;
b16 halogensubstituierte C₂-C₂₀-Olefine wie Vinylchlorid, Vinylidenchlorid, Vinylbromid, Fluorethen, 1,1-Difluorethen und Tetrafluorethen;
b17 monoethylenisch ungesättigte Monomere, die eine oder zwei Epoxidgruppen aufweisen, wie Mono- und Diester von monoethylenisch ungesättigten Mono- oder Dicarbonsäuren, insbesondere Mono- und Diester von C₃-C₁₀-Epoxyalkanolen, z. B. Mono- oder Diglycidylester monoethylenisch ungesättigter C₃-C₈-Mono- oder C₄-C₈-Dicarbonsäuren wie Glycidylacrylat und Glycidylmethacrylat, oder monoethylenisch ungesättigte Ether von C₃-C₁₀-Epoxyalkanolen, insbesondere Allyl- oder Methallylether, z. B. Allylglycidylether und Methallylglycidylether;
b18 monoethylenisch ungesättigte Monomere, die wenigstens eine Carbonatgruppe, insbesondere eine cyclische Carbonatgruppe, z. B. eine 1,3-Dioxolan-2-on-Gruppe oder 4-Methyl-1,3-dioxolan-2-on-Gruppe, aufweisen, z. B. Propylencarbonat-acrylat ([1,3-Dioxolan-2-on-4-yl]methylacrylat) oder Propylencarbonatmethacrylat ([1,3-Dioxolan-2-on-4-yl]methylmethacrylat);
b19 Ester monoethylenisch ungesättigter C₃-C₈-Monocarbonsäuren oder monoethylenisch ungesättigter C₄-C₈-Dicarbonsäuren mit C₈-C₂₄-Alkenolen oder C₈-C₂₄-Alkandienolen, insbesondere die Ester der Acrylsäure oder der Methacrylsäure, wie beispielsweise Oleylacrylat, Oleylmethacrylat, Linolylacrylat oder Linolylmethacrylat.

Bevorzugte Comonomere b sind die Monomere der Gruppen b1, b2, b4, b5, b6, b8, b9, b10, b12 und b13, insbesondere die Monomere der Gruppen b9 wie vorzugsweise vinylaromatische Kohlenwasserstoffe, speziell Styrol, und b10, vorzugsweise Ester der Acrylsäure oder Methacrylsäure mit C₁-C₂₀-Alkoholen, sowie Kombinationen der Monomere b1, b2, b4, b5, b6, b8, b9, b10, b12 und b13, insbesondere b9 und/oder b10, mit einem oder mehreren Monomeren der Gruppe b17, b18 oder b19.

Sofern die Monomere der Formel I copolymerisiert werden, handelt es sich bei den Comonomeren bevorzugt um ein oder mehrere Comonomere, die unter den Monomeren der Gruppen b9 und b10 ausgewählt sind, wie vorzugsweise vinylaromatische Kohlenwasserstoffe, speziell Styrol, und Ester der Acrylsäure oder Methacrylsäure mit C₁ C₂₀-Alkoholen, wie n-Butylacrylat, 2-Ethylhexylacrylat, Methylacrylat und Methylmethacrylat.

Sofern das erfindungsgemäße Polymer wenigstens ein Comonomer b, vorzugsweise ein Comonomer der Gruppen b9 und b10, einpolymerisiert enthält, umfassen die zu polymerisierenden Monomere M in der Regel 1 bis 99 Gew.-%, insbesondere 5 bis 95 Gew.-% und speziell 10 bis 90 Gew.-% wenigstens einer Verbindung der Formel I und 1 bis 99 Gew.-%, insbesondere 5 bis 95 Gew.-% und speziell 10 bis 90 Gew.-% wenigstens eines, vorzugsweise monoethylenisch ungesättigten, Comonomers b, wobei die Angaben in Gew.-% auf die Gesamtmenge der Monomere M bezogen sind. Dementsprechend machen die Wiederholungseinheiten der Formel I" 1 bis 99 Gew.-%, insbesondere 5 bis 95 Gew.-% und speziell 10 bis 90 Gew.-% und die von den Comonomeren b abgeleiteten Wiederholungseinheiten 1 bis 99 Gew.-%, insbesondere 5 bis 95 Gew.-% und speziell 10 bis 90 Gew.-%, bezogen auf das Gesamtgewicht aller Wiederholungseinheiten, aus.

Sofern die Monomere der Formel I mit einem Comonomer der Gruppe b9, wie einem vinylaromatischen Kohlenwasserstoff wie beispielsweise Styrol, und einem Comonomer der Gruppe b10, wie einem Ester der Acrylsäure oder Methacrylsäure mit einem C₁-C₂₀-Alkohol wie beispielsweise Methylacrylat, Methylmethacrylat, n-Butylacrylat oder 2-Ethylhexylacrylat, copolymerisiert werden, setzt man die zu polymerisierenden Monomere der Gruppe b9 in der Regel in einer Menge von 10 bis 80 Gew.-%, insbesondere 20 bis 60 Gew.-%, und die zu polymerisierenden Monomere der Gruppe b10 in einer Menge von 10 bis 80 Gew.-%, insbesondere 20 bis 60 Gew.-%, ein, wobei die Angaben in Gew.-% auf die Gesamtmenge der Monomere M bezogen sind. Die Monomere der Formel I machen dann vorzugsweise 10 bis 80 Gew.-%, insbesondere 20 bis 60 Gew.-%, bezogen auf die Gesamtmenge der Monomere M, aus. Insbesondere setzt man dann die Comonomere der Gruppen b9 und b10 im Verhältnis von Comonomeren der Gruppe b9 zu Comonomeren der Gruppe b10 von 10 : 1 bis 1 : 10, insbesondere 5 : 1 bis 1 : 5 ein.

Sofern die Monomere der Formel I mit einem Comonomer der Gruppe b10, wie einem Ester der Acrylsäure oder Methacrylsäure mit einem C₁-C₂₀-Alkohol wie beispielsweise Methylacrylat, Methylmethacrylat, n-Butylacrylat oder 2-Ethylhexylacrylat, copolymerisiert werden, setzt man die zu polymerisierenden Monomere der Gruppe b10 in der Regel in einer Menge von 10 bis 95 Gew.-%, vorzugsweise 20 bis 90 Gew.-% und die Monomere der Formel I in einer Menge von 5 bis 90 Gew.-%, vorzugsweise 10 bis 80 Gew.-%, bezogen auf die Gesamtmenge der Monomere M, ein.

In einer bevorzugten Ausführungsform umfassen die Comonomere b wenigstens 60 Gew.-%, vorzugsweise wenigstens 80 Gew.-%, insbesondere wenigstens 90 Gew.-%, bezogen auf die Gesamtmenge der Comonomere b, wenigstens eines hydrophoben Monomers, das eine Wasserlöslichkeit von nicht mehr als 80 g/L bei 25 °C aufweist. Beispiele für hydrophobe Monomere b sind die Comonomere der Gruppen b9 bis b16 und hierunter bevorzugt die Comonomere der Gruppen b9, b10, b12, b13 und b14.

In einer weiteren Ausführungsform umfassen die Comonomere b 60 bis 99,99 Gew.-%, vorzugsweise 80 bis 99,95 Gew.-%, insbesondere 90 bis 99,9 Gew.-%, bezogen auf die Gesamtmenge der Comonomere b, wenigstens eines hydrophoben Monomers, das eine Wasserlöslichkeit von nicht mehr als 80 g/L bei 25 °C aufweist und 0,01 bis 40 Gew.-%, insbesondere 0,05 bis 20 Gew.-% oder 0,1 bis 10 Gew.-%, bezogen auf die Gesamtmenge der Comonomere b, wenigstens eines hydrophilen Monomers, das eine Wasserlöslichkeit von mehr als 80 g/L bei 25 °C aufweist. Beispiele für hydrophobe Monomere b sind die Comonomere der Gruppen b9 bis b17 und b19 und hierunter bevorzugt die Comonomere der Gruppen b9, b10, b12, b13 und b14. Beispiele für hydrophile Monomere b sind die Comonomere der Gruppen b1 bis b8 und b18 und hierunter bevorzugt die Comonomere der Gruppen b1, b2, b4, b5, b6 und b8.

Außerdem kann es zweckmäßig sein, dass die Monomere M neben den Monomeren der Formel I und gegebenenfalls dem bzw. den Comonomer(en) b ein oder mehrere mehrfach ethylenisch ungesättigte Monomere mit z. B. 2, 3 oder 4 nicht konjugierten, ethylenisch ungesättigten Doppelbindungen umfassen, die im Folgenden auch als Monomere c bezeichnet werden. Beispiele für Monomere c sind Diester und Triester ethylenisch ungesättigter Carbonsäuren, insbesondere die Bis- und Trisacrylate von Diolen oder Polyolen mit 3 oder mehr OH-Gruppen, z. B. die Bisacrylate und die Bismethacrylate von Ethylenglykol, Diethylenglykol, Triethylenglykol, Neopentylglykol oder Polyethylenglykolen. Derartige Monomere c werden, sofern erwünscht, in der Regel in einer Menge von 0,01 bis 10 Gew.-%, bezogen auf die Gesamtmenge der zu polymerisierenden Monomere M, eingesetzt.

Die erfindungsgemäßen Polymere weisen in der Regel ein zahlenmittleres Molekulargewicht im Bereich von 1000 bis 10⁶ g/mol, insbesondere im Bereich von 1200 bis 10⁵ g/mol auf. Das gewichtsmittlere Molekulargewicht der erfindungsgemäßen Polymere liegt häufig im Bereich von 1200 bis 5x10⁶ g/mol, insbesondere im Bereich von 2000 bis 2x10⁶ g/mol.

Die Polymerisation der Monomere M kann nach üblichen Verfahren der radikalischen Polymerisation durchgeführt werden. Hierzu zählen Lösungs- und Fällungspolymerisation, Suspensionspolymerisation und Emulsionspolymerisation, einschließlich einer Miniemulsionspolymerisation.

In einer bevorzugten Ausführungsform der Erfindung erfolgt das Polymerisationsverfahren in einem nicht-wässrigen Lösungs- oder Verdünnungsmittel als Polymerisationsmedium. Mit anderen Worten, die Polymerisation wird in einem Lösungs- oder Verdünnungsmittel im Sinne einer Lösungs- oder Fällungspolymerisation durchgeführt, das kein Wasser oder nur geringe Mengen an Wasser enthält. Bezogen auf das Gesamtvolumen der Polymerisationsmischung beträgt die Menge an Wasser häufig nicht mehr als 2 Gew.-%, insbesondere nicht mehr als 1 Gew.-% und speziell nicht mehr als 0,5 Gew.-%. Typischerweise liegt die Menge an Wasser, bezogen auf das Monomer, bei nicht mehr als 10 Gew.-%, häufig nicht mehr als 5 Gew.-%, insbesondere nicht mehr als 2 Gew.-% und speziell nicht mehr als 1 Gew.-%.

Geeignete Lösungs- oder Verdünnungsmittel sind insbesondere solche, in dem die zu polymerisierenden Monomere M löslich sind. Man kann auch in organischen Lösungsmitteln polymerisieren, in denen die zu polymerisierenden Monomere nicht löslich sind. Die Polymerisation erfolgt dann als eine ÖI-in-ÖI-Emulsions- oder Suspensionspolymerisation, wobei, abhängig von den Mengenverhältnissen von Monomeren und organischem Lösungsmittel, die Monomere die kohärente Phase oder vorzugsweise die disperse Phase bilden.

Geeignete Lösungsmittel umfassen insbesondere aprotische Lösungsmittel. Hierzu zählen aliphatische und cycloaliphatische Kohlenwasserstoffe und Halogenkohlenwasserstoffe wie n-Hexan, n-Heptan, Cyclohexan, Dichlormethan, 1,2-Dichlorethan, aromatische Kohlenwasserstoffe und aromatische Halogenkohlenwasserstoffe wie Benzol, Toluol, Xylole, Chlorbenzol, Dichlorbenzole, Anhydride aliphatischer, nicht-polymerisierbarer Carbonsäuren wie Acetanhydrid, C₁-C₆-Alkylester und C₅-C₆-Cycloalkylester aliphatischer Monocarbonsäuren mit 1 bis 4 C-Atomen wie Methylacetat, Ethylacetat, Propylacetat, n-Butylacetat, Methylbutyrat, Ethylbutyrat, Propylbutyrat, Methylpropionat, Ethylpropionat, Propylpropionat, Ethylformiat, Butylformiat, Cyclohexylacetat und dergleichen, C₁-C₄-Alkoxy-C₂-C₄-alkylalkanoate wie 1-Methoxy-2-propylacetat oder 2-Methoxyethylacetat, N,N-Di-C₁-C₄-alkylamide von aliphatischen C₁-C₄-Carbonsäuren wie N,N-Dimethylformamid, N,N-Dimethylacetamid, N-C₁-C₄-Alkyllactame wie N-Methylpyrrolidon, N-Ethylpyrrolidon, Di-C₁-C₄-alkylsulfoxide wie Dimethylsulfoxid, alicyclische und cyclische Ketone mit 3 bis 8 C-Atomen wie Methylethylketon, Aceton und Cyclohexanon, Di-C₁-C₄-alkylether und aliphatische, cycloaliphatische und aromatische Ether wie Diethylether, Methyl-tert.-butylether, Dioxan, Tetrahydrofuran, Monoglyme und Anisol, außerdem cyclische und acyclische, gesättigte Carbonate mit vorzugsweise 3 bis 8 C-Atomen wie Ethylencarbonat (1,3-Dioxolan-2-on) und Propylencarbonat, C₁-C₄-Dialkylcarbonate wie Dimethylcarbonat, Diethylcarbonat, Dipropylcarbonat, Dibutylcarbonat sowie Gemische der vorgenannten aprotischen Lösungsmittel. Geeignete Lösungsmittel für die Polymerisation sind außerdem protische Lösungsmittel und deren Gemische mit einem oder mehreren aprotischen Lösungsmitteln. Hierzu zählen vor allem aliphatische Alkohole wie C₂-C₄-Alkylenglykolmono-C₁-C₄-alkylether wie 1-Methoxy-2-propanol, C₁-C₄-Alkyl- C₂-C₄-alkylenglykolmono-C₁-C₄-alkylether wie 1-Methoxy-2-methyl-2-propanol, C₁-C₁₀-Alkanole wie Methanol, Ethanol, n-Propanol, Isopropanol, n-Butanol, 2-Butanol, Isobutanol, tert.-Butanol, Amylalkohol, Isoamylalkohol sowie Gemische der vorgenannten protischen Lösungsmittel.

Bevorzugte Lösungsmittel sind C₁-C₆-Alkylester von aliphatischen C₁-C₄-Monocarbonsäuren wie n-Butylacetat, C₂-C₄-Alkylenglykolmono-C₁-C₄-alkylether wie 1-Methoxy-2-propanol, C₁-C₄-Alkyl- C₂-C₄-alkylenglykolmono-C₁-C₄-alkylether wie 1-Methoxy-2-methyl-2-propanol, C₁-C₄-Dialkylcarbonate wie Dimethylcarbonat, Diethylcarbonat, Dipropylcarbonat, Dibutylcarbonat, cyclische Carbonate wie Ethylencarbonat und Propylencarbonat, Ether wie Glyme und Anisol.

Im Falle der Fällungspolymerisation handelt es sich um ein organisches Lösungsmittel oder Verdünnungsmittel, in dem das Copolymer unlöslich ist. Im Falle der Lösungspolymerisation handelt es sich typischerweise um ein organisches Lösungsmittel, in dem das Copolymer löslich ist.

In der Regel wird man das organische Lösungsmittel so bemessen, dass die Menge der zu polymerisierenden Monomere M, bezogen auf die Gesamtmenge an Monomeren M plus Lösungsmittel, im Bereich von 10 bis 65 Gew.-%, insbesondere im Bereich von 20 bis 60 Gew.-% liegt. Bei einer Lösungspolymerisation werden dementsprechend Polymerlösungen mit Feststoffgehalten im Bereich von 10 bis 90 Gew.-% und insbesondere 20 bis 80 Gew.-% erhalten.

Die Polymerisation der Monomere M kann nach üblichen Verfahren der radikalischen Homo- oder Copolymerisation erfolgen. In der Regel wird man hierzu die Monomere M unter Reaktionsbedingungen polymerisieren, bei denen sich Radikale bilden.

Die Bildung der Radikale erfolgt in der Regel durch Einsatz eines sogenannten Polymerisationsinitiators, d. h. einer Verbindung, die beim Zerfall, der chemisch, thermisch oder photochemisch ausgelöst werden kann, Radikale bildet.

Zu den geeigneten Polymerisationsinitiatoren zählen organische Azoverbindungen, organische Peroxide und Hydroperoxide, anorganische Peroxide und sogenannte Redoxinitiatoren. Zu den organischen Peroxidverbindungen zählen beispielsweise tert.-Butylperoxy-2-ethylhexanoat, tert.-Butylperoxypivalat, Acetylperoxid, Benzoylperoxid, Lauroylperoxid, tert.-Butylperoxyisobutyrat, Caproylperoxid. Zu den Hydroperoxiden zählen neben Wasserstoffperoxid auch organische Hydroperoxide wie Cumolhydroperoxid, tert.-Butylhydroperoxid, tert.-Amylhydroperoxid und dergleichen. Zu den Azoverbindungen zählen beispielsweise 2,2'-Azobis-isobutyronitril, 2,2'-Azobis(2-methylbutyronitril), 2,2'-Azobis[2-methyl-N-(2-hydroxyethyl)propionamid], 1,1'-Azobis(1-cyclohexancarbonitril), 2,2'-Azobis(2,4-dimethylvaleronitril), 2,2'-Azobis(N,N'-dimethylenisobutyroamidin) 2,2'-Azobis(N,N'-dimethylenisobutyroamidin), 2,2'-Azo-bis(2-methylpropionamidin), N-(3-Hydroxy-1,1-bis-hydroxymethylpropyl)-2-[1-(3-hydroxy-1,1-bis-hydroxymethyl-propylcarbamoyl)-1-methyl-ethylazo]-2-methyl-propionamid sowie N-(1-Ethyl-3-hydroxypropyl)-2-[1-(1-ethyl-3-hydroxypropylcarbamoyl)-1-methyl-ethylazo]-2-methyl-propionamid. Zu den anorganischen Peroxiden zählen Peroxodischwefelsäure und deren Salze wie Ammonium-, Natrium- und Kaliumperoxodisulfat. Unter Redoxinitiatorsystemen versteht man Initiatorsysteme, die ein Oxidationsmittel, beispielsweise ein Salz der Peroxodischwefelsäure, Wasserstoffperoxid oder ein organisches Peroxid wie tert.-Butylhydroperoxid, und ein Reduktionsmittel enthalten. Als Reduktionsmittel enthalten sie vorzugsweise eine Schwefelverbindung, die insbesondere ausgewählt ist unter Natriumhydrogensulfit, Natriumhydroxymethansulfinat und dem Hydrogensulfit-Addukt an Aceton. Weitere geeignete Reduktionsmittel sind phosphorhaltige Verbindungen wie phosphorige Säure, Hypophosphite und Phosphinate sowie Hydrazin bzw. Hydrazinhydrat und Ascorbinsäure. Weiterhin können Redoxinitiatorsysteme einen Zusatz geringer Mengen von Redoxmetallsalzen wie Eisensalze, Vanadiumsalze, Kupfersalze, Chromsalze oder Mangansalze enthalten, wie beispielsweise das Redoxinitiatorsystem Ascorbinsäure/Eisen(II)sulfat/Natriumperoxodisulfat. Besonders bevorzugte Initiatoren für das erfindungsgemäße Polymerisationsverfahren sind Azoverbindungen, speziell Azobisisobutyronitril (AIBN).

Zur radikalischen Polymerisation der Monomere M werden diese Polymerisationsinitiatoren in der Regel in einer Menge von 0,01 bis 5 Gew.-%, insbesondere in einer Menge von 0,1 bis 3 Gew.-%, bezogen auf die zu polymerisierenden Monomere, eingesetzt.

Zur Polymerisation können die üblichen Polymerisationstechniken angewendet werden. Hier sind insbesondere ein (Semi-)Batch-Verfahren, bei dem die Hauptmenge, d. h. wenigstens 60 Gew.-%, insbesondere wenigstens 80 Gew.-% und häufig die Gesamtmenge der zu polymerisierenden Monomere M im Polymerisationsgefäß vorgelegt wird, sowie das Monomerzulaufverfahren, bei dem die Hauptmenge der zu polymerisierenden Monomere M, häufig wenigstens 60 Gew.-%, insbesondere wenigstens 80 Gew.-% und speziell wenigstens 90 Gew.-%, im Verlauf der Polymerisationsreaktion in das Polymerisationsgefäß gegeben wird, zu nennen. Aus Praktikabilitätsgründen wird bei größeren Ansätzen die Polymerisation häufig als Monomerzulaufverfahren durchgeführt.

Der Polymerisationsinitiator kann im Polymerisationsgefäß vorgelegt oder im Verlauf der Polymerisationsreaktion zugegeben werden. Häufig wird man so vorgehen, dass man wenigstens einen Teil des Initiators, vorzugsweise wenigstens 50 Gew.-% und insbesondere wenigstens 80 Gew.-% des Polymerisationsinitiators, im Verlauf der Polymerisationsreaktion zugibt.

Insbesondere hat es sich bewährt, einen geringen Teil der Monomere M, z. B. 0,1 bis 20 Gew.-%, bezogen auf die Gesamtmenge der zu polymerisierenden Monomere M, gegebenenfalls zusammen mit einer Teilmenge oder der Gesamtmenge an Polymerisationsinitiator und einem Teil oder der Gesamtmenge des Lösungs- bzw. Verdünnungsmittels, im Polymerisationsgefäß vorzulegen, die Polymerisation zu starten, beispielsweise durch Erwärmen der Polymerisationsmischung, und dann die Restmenge der Monomere M und, sofern erforderlich, die Restmenge an Polymerisationsinitiator und Lösungsmittel im Verlauf der Polymerisation zuzugeben.

Die für die Polymerisation üblicherweise angewendeten Polymerisationstemperaturen liegen, abhängig von dem gewählten Initiatorsystem, in der Regel im Bereich von 20 bis 180 °C, insbesondere im Bereich von 40 bis 130 °C und speziell im Bereich von 50 bis 120 °C.

Der Polymerisationsdruck ist von untergeordneter Bedeutung und kann im Bereich von Normaldruck oder leichtem Unterdruck, z. B. > 800 mbar, oder bei Überdruck, z. B. bis 10 bar, liegen, wobei höhere oder niedrigere Drücke ebenfalls angewendet werden können.

Die Polymerisationsdauer wird in der Regel 10 Stunden nicht überschreiten und liegt häufig im Bereich von 1 bis 8 Stunden.

Das erfindungsgemäße Polymerisationsverfahren kann in den für eine radikalische Polymerisation üblichen Reaktoren durchgeführt werden, beispielsweise Rührkesseln, insbesondere solchen mit wandgängigen Rührern, einschließlich Rührkesselkaskaden sowie Rohrreaktoren, die gegebenenfalls dynamische und/oder statische Mischelemente aufweisen können. Die Reaktoren weisen in der Regel ein oder mehrere Vorrichtungen zur Zuführung der Edukte und Vorrichtungen zur Entnahme der Produkte sowie gegebenenfalls Mittel zur Zufuhr und zur Abfuhr der Reaktionswärme sowie gegebenenfalls Mittel zur Steuerung und/oder Kontrolle der Reaktionsparameter Druck, Temperatur, Umsatz etc. auf. Die Reaktoren können absatzweise oder kontinuierlich betrieben werden.

Nach Beendigung der Polymerisation kann man das Polymerisationsgemisch in üblicher Weise aufarbeiten. Im Falle einer Fällungspolymerisation kann man das Polymer beispielsweise abfiltrieren. Flüchtige Komponenten, beispielsweise Lösungsmittel, lassen sich auch durch destillative Maßnahmen abtrennen. Im Falle einer Lösungspolymerisation kann man auch eine Fällung des erhaltenen Polymerisats herbeiführen, beispielsweise durch Zugabe eines organischen Lösungsmittels, in welchem das Polymerisat nicht löslich ist. Gegebenenfalls kann man im Anschluss an die Polymerisation auch einen Lösungsmittelaustausch durchführen, z. B. um das Polymer von einer Lösung in eine Dispersion zu überführen. Gegebenenfalls wird man das erhaltene Polymerisat einer Entgasung unterziehen, um weitere flüchtige Bestandteile zu entfernen.

Die erfindungsgemäßen Polymere eignen sich als Komponente in 2K-Bindemittelzusammensetzungen.

Unter 2K-Bindemittelzusammensetzungen versteht man ein Bindemittel, das wenigstens zwei polyfunktionelle Bindemittelbestandteile enthält, die miteinander unter Bindungsbildung reagieren und dabei ein polymeres Netzwerk ausbilden. Die erfindungsgemäßen Polymere können aufgrund der darin vorhandenen Alkyliden-1,3-dioxolan-2-on-Gruppen mit zahlreichen nucleophilen Gruppen unter Bindungsbildung reagieren. Beispiele für solche nucleophilen Gruppen sind vor allem aliphatische Hydroxylgruppen, aliphatische primäre und sekundäre Aminogruppen, Phosphingruppen, insbesondere aliphatische Phosphingruppen, Phosphonatgruppen, insbesondere aliphatische Phosphonatgruppen, sowie analoge Phosphorverbindungen, außerdem Mercaptangruppen, insbesondere aliphatische Mecaptangruppen.

Dementsprechend enthalten 2K-Bindemittelzusammensetzungen neben wenigstens einem erfindungsgemäßen Polymer in der Regel zusätzlich wenigstens eine Verbindung, die wenigstens 2 funktionelle Gruppen F, z. B. 2, 3, 4, 5, 6, 7, 8, 9 oder 10 funktionelle Gruppen F, aufweist, welche unter aliphatischen Hydroxylgruppen, aliphatischen primären oder sekundären Aminogruppen, aliphatischen Phosphin-, Phosphonat- u. ä. Gruppen und aliphatischen Mercaptangruppen ausgewählt sind. Diese Verbindungen werden im Folgenden auch als Härter bezeichnet.

In der Regel wird die Menge an Härter so gewählt, dass das Molverhältnis von funktionellen Alkyliden-1,3-dioxolan-2-on-Gruppen der Formel I' zu den funktionellen Gruppen F im Härter im Bereich von 1 : 10 bis 10 : 1, insbesondere im Bereich von 5 : 1 bis 1 : 5 und speziell im Bereich von 1 : 2 bis 2 : 1 liegt.

Bevorzugte funktionelle Gruppen F sind aliphatische Hydroxylgruppen und aliphatische primäre und sekundäre Aminogruppen.

Der Härter kann eine niedermolekulare Substanz sein, d. h., sein Molekulargewicht liegt unterhalb 500 g/mol, oder eine oligomere oder polymere Substanz, die ein zahlenmittleres Molekulargewicht oberhalb 500 g/mol aufweist.

Die 2K-Bindemittelzusammensetzungen können auch einen oder mehrere geeignete Katalysatoren für die Aushärtung enthalten, die sich in bekannter Weise nach der Art der reaktiven funktionellen Gruppen F richten. Die Katalysatoren werden, sofern erwünscht, in Anteilen von 0,01 Gew.-% bis etwa 10 Gew.-%, bezogen auf das Gesamtgewicht der erfindungsgemäßen Polymere mit funktionellen Gruppen der Formel I' und des Härters, eingesetzt. In einer Ausgestaltung werden keine Katalysatoren benötigt, insbesondere bei Härtern, die als funktionelle Gruppen Aminogruppen aufweisen, d. h., der Gehalt an Katalysatoren in der Zusammensetzung beträgt dann weniger als 0,01 Gew.-%. Katalysatoren werden bevorzugt dann eingesetzt, wenn der Härter reaktive Gruppen F aufweist, die von Aminogruppen verschieden sind, insbesondere wenn der Härter Hydroxylgruppen aufweist.

Bevorzugt eingesetzte Katalysatoren sind basische Katalysatoren, besonders bevorzugt organische Amine und organische Phosphine. Unter den organischen Aminen sind Amidinbasen, wie beispielsweise 1,8-Diazabicyclo[5.4.0]undec-7-en (DBU) und 1,5-Diazabicyclo[4.3.0]non-5-en (DBN), Mono-C₁-C₆-alkyl-, Di-C₁-C₆-alkyl- und Tri-C₁-C₆-alkylamine, insbesondere Triethylamin und tert.-Butylamin, bevorzugt. Unter den organischen Phosphinen sind Trialkylphosphine und Triarylphosphine bevorzugt, beispielsweise Tri-n-butylphosphin und Triphenylphosphin. Die Katalysatoren können selbstverständlich auch als Mischungen eingesetzt werden, gegebenenfalls in Kombination mit Tri-C₁-C₆-alkylammoniumhalogeniden und Kupfersalzen, zum Beispiel Triphenylphosphin in Kombination mit einem Tri-C₁-C₆-alkylammoniumhalogenid und einem Kupfersalz, z. B. Kupfer(I)-chlorid, Kupfer(I)-bromid, Kupfer(II)-chlorid oder Kupfer(II)-sulfat.

Zu den erfindungsgemäß bevorzugten Härtern gehören aminische Härter, d. h. Härter, die wenigstens zwei primäre oder sekundäre Aminogruppen aufweisen, und alkoholische Härter, also Verbindungen, die wenigstens zwei Hydroxylgruppen aufweisen.

Zu den aminischen Härtern, im Folgenden auch Aminhärter, zählen beispielsweise aliphatische und cycloaliphatische Polyamine, aromatische und araliphatische Polyamine sowie polymere Amine, z. B. Aminoplaste und Polyamidoamine. Aminhärter vernetzen Polymere mit 1,3-Dioxolan-2-on-Gruppen, im Folgenden auch Carbonatpolymere genannt, durch Reaktion der primären oder sekundären Aminofunktionen der Polyamine mit den 1,3-Dioxolan-2-on-Gruppen der Carbonatpolymere unter Ausbildung von Urethanfunktionen. Bevorzugte Polyaminhärter weisen im Mittel wenigstens zwei primäre oder sekundäre Aminogruppen pro Molekül, z. B. zwei, drei oder vier primäre oder sekundäre Aminogruppen pro Molekül, auf. Sie können auch zusätzlich ein oder mehrere tertiäre Aminogruppen enthalten. Geeignete Polyamine sind beispielsweise
- aliphatische Polyamine wie Ethylendiamin, 1,2- und 1,3-Propandiamin, Neopentandiamin, Hexamethylendiamin, Octamethylendiamin, 1,10-Diaminodecan, 1,12-Diaminododecan, Diethylentriamin, Triethylentetramin, Tetraethylenpentamin, 2,2-Dimethylpropylendiamin, Trimethylhexamethylendiamin, 1-(3-Aminopropyl)-3-aminopropan, 1,3-Bis-(3-aminopropyl)propan, 4-Ethyl-4-methylamino-1-octylamin und dergleichen;
- cycloaliphatische Diamine wie 1,2-Diaminocyclohexan, 1,2-, 1,3-, 1,4-Bis(aminomethyl)cyclohexan, 1-Methyl-2,4-diaminocyclohexan, N-Cyclohexylpropylen-1,3-diamin, 4-(2-Aminopropan-2-yl)-1-methylcyclohexan-1-amin, Isophorondiamin, 4,4'-Diaminodicyclohexylmethan (Dicykan), 3,3'-Dimethyl-4,4'-diaminodicyclohexylmethan, 3,3',5,5'-Tetramethyl-4,4'-diaminodicyclohexylmethan, 4,8-Diamino-tricyclo[5.2.1.0]decan, Norbornandiamin, Menthandiamin, Menthendiamin und dergleichen;
- aromatische Diamine wie Toluylendiamin, Xylylendiamin, insbesondere meta-Xylylendiamin (MXDA), Bis(4-aminophenyl)methan (MDA oder Methylendianilin), Bis(4-aminophenyl)sulfon (auch als DADS, DDS oder Dapson bekannt) und dergleichen;
- cyclische Polyamine wie Piperazin, N-Aminoethylpiperazin und dergleichen;
- Polyetheramine, insbesondere difunktionelle und trifunktionelle primäre Polyetheramine auf der Basis von Polypropylenglykol, Polyethylenglykol, Polybutylenoxid, Poly-(1,4-butandiol), Poly-Tetrahydrofuran (Poly-THF) oder Polypentylenoxid, z. B. 4,7,10-Trioxatridecan-1,3-diamin, 4,7,10-Trioxatridecan-1,13-diamin, 1,8-Diamino-3,6-dioxaoctan (XTJ-504, Fa. Huntsman), 1,10-Diamino-4,7-dioxadecan (XTJ-590, Fa. Huntsman), 1,12-Diamino-4,9-dioxadodecan (Fa. BASF SE), 1,3-Diamino-4,7,10-trioxatridecan (Fa. BASF SE), primäre Polyetheramine auf der Basis von Polypropylenglykol mit einer mittleren Molmasse von 230 wie z. B. Polyetheramine D 230 (Fa. BASF SE) oder Jeffamine^{®} D 230 (Fa. Huntsman), difunktionelle, primäre Polyetheramine auf der Basis von Polypropylenglykol mit einer mittleren Molmasse von 400, z. B. Polyetheramine D 400 (Fa. BASF SE) oder Jeffamine^{®} XTJ 582 (Fa. Huntsman), difunktionelle, primäre Polyetheramine auf Basis von Polypropylenglykol mit einer mittleren Molmasse von 2000 wie z. B. Polyetheramine D 2000 (Fa. BASF SE), Jeffamine^{®} D2000 oder Jeffamine^{®} XTJ 578 (jeweils Fa. Huntsman), difunktionelle, primäre Polyetheramine auf der Basis von Propylenoxid mit einer mittleren Molmasse von 4000 wie z. B. Polyetheramin D 4000 (Fa. BASF SE), trifunktionelle, primäre Polyetheramine hergestellt durch Reaktion von Propylenoxid mit Trimethylolpropan, gefolgt durch eine Aminierung der endständigen OH-Gruppen mit einer mittleren Molmasse von 403 wie z. B. Polyetheramine T 403 (Fa. BASF SE) oder Jeffamine^{®} T 403 (Fa. Huntsman), trifunktionelle, primären Polyetheramin, hergestellt durch Reaktion von Propylenoxid mit Glycerin, gefolgt durch eine Aminierung der endständigen OH-Gruppen mit einer mittleren Molmasse von 5000 wie z. B. Polyetheramine T 5000 (Fa. BASF SE) oder Jeffamine^{®} T 5000 (Fa. Huntsman), aliphatische Polyetheramine, die aus einem mit Propylenoxid gepfropften Polyethylenglykol aufgebaut sind und eine mittlere Molmasse von 600 aufweisen, wie z. B. Jeffamine^{®} ED-600 bzw. Jeffamine^{®} XTJ-501 (jeweils Fa. Huntsman), aliphatische Polyetheramine, die aus einem mit Propylenoxid gepfropften Polyethylenglykol aufgebaut sind und eine mittlere Molmasse von 900 aufweisen, wie z. B. Jeffamine^{®} ED-900 (Fa. Huntsman), aliphatische Polyetheramine, die aus einem mit Propylenoxid gepfropften Polyethylenglykol aufgebaut sind und eine mittlere Molmasse von 2000 aufweisen, wie z. B. Jeffamine^{®} ED-2003 (Fa. Huntsman), difunktionelle, primäre Polyetheramin, hergestellt durch Aminierung eines mit Propylenoxid gepfropften Diethylenglykols mit einer mittleren Molmasse von 220 wie z. B. Jeffamine^{®} HK-511 (Fa. Huntsman), aliphatische Polyetheramine auf der Basis eines Copolymers aus Poly-(tetramethylenetherglykol) und Polypropylenglykol mit einer mittleren Molmasse von 1000 wie z. B. Jeffamine^{®} XTJ-542 (Fa. Huntsman), aliphatische Polyetheramine auf der Basis eines Copolymers aus Poly(tetramethylenetherglykol) und Polypropylenglykol mit einer mittleren Molmasse von 1900 wie z. B. Jeffamine^{®} XTJ-548 (Fa. Huntsman), aliphatische Polyetheramine auf der Basis eines Copolymers aus Poly(tetramethylenetherglykol) und Polypropylenglykol mit einer mittleren Molmasse von 1400 wie z. B. Jeffamine^{®} XTJ-559 (Fa. Huntsman), Polyethertriamine auf der Basis eines mit Butylenoxid gepfropften mindestens dreiwertigen Alkohols mit einer mittleren Molmasse von 400 wie z. B. Jeffamine^{®} XTJ-566 (Fa. Huntsman), aliphatische Polyetheramine, hergestellt durch Aminierung von mit Butylenoxid aufgepfropften Alkoholen mit einer mittleren Molmasse von 219 wie z. B. Jeffamine^{®} XTJ-568 (Fa. Huntsman), Polyetheramine auf der Basis von Pentaerythrit und Propylenoxid mit einer mittleren Molmasse von 600 wie z. B. Jeffamine^{®} XTJ-616 (Fa. Huntsman), Polyetheramine auf der Basis von Triethylenglykol mit einer mittleren Molmasse von 148, z. B. Jeffamine^{®} EDR-148 (Fa. Huntsman), difunktionelle, primäre Polyetheramine, hergestellt durch Aminierung eines mit Propylenoxid gepfropften Ethylenglykols mit einer mittleren Molmasse von 176 wie z. B. Jeffamine^{®} EDR-176 (Fa. Huntsman) sowie Polyetheramine, hergestellt durch Aminierung von Poly-Tetrahydrofuran (Poly-THF) mit einer mittleren Molmasse von 250, z. B. PolyTHF-Amin 350 (Fa. BASF SE) und Mischungen dieser Amine;
- Polyamidoamine (Amidopolyamine), die durch die Reaktion von dimeren Fettsäuren (z. B. dimere Linolsäure) mit niedermolekularen Polyaminen wie Diethylentriamin, 1-(3-Aminopropyl)-3-aminopropan oder Triethylentetramin oder anderen Diaminen wie den zuvor genannten aliphatischen oder cycloaliphatischen Diaminen erhältlich sind;
- Addukte, die durch Umsetzung von Aminen, insbesondere Diaminen, mit einem Unterschuss an Epoxidharz bzw. Reaktivverdünner erhältlich sind, wobei man vorzugsweise solche Addukte einsetzt, worin etwa 5 bis 20 % der Epoxidgruppen mit Aminen, insbesondere Diaminen, umgesetzt worden sind;
- Phenalkamine, wie aus der Epoxidchemie bekannt;
- Mannichbasen, welche z. B. durch Kondensation von Polyaminen, vorzugsweise Diethylentriamin, Triethylentetramin, Isophorondiamin, 2,2,4- bzw. 2,4,4-Trimethylhexamethylendiamin, 1,3- und 1,4-Bis(aminomethyl)cyclohexan mit Aldehyden, vorzugsweise Formaldehyd und ein- oder mehrwertigen Phenolen mit mindestens einer aldehydreaktiven Kernstelle, z. B. die verschiedenen Kresole und Xylenole, p-tert.-Butylphenol, Resorcin, 4,4'-Dihydroxydiphenylmethan, 4,4'-Dihydroxydiphenyl-2,2-propan, vorzugsweise aber Phenol, hergestellt werden;
sowie Mischungen der vorgenannten Aminhärter, insbesondere Mischungen von difunktionellen Aminen aus der Gruppe der aliphatischen, cycloaliphatischen und aromatischen Amine mit den vorgenannten Polyetheraminen.

Bevorzugte aminische Härter sind aliphatische Polyamine, insbesondere 2,2-Dimethylpropylendiamin, aromatische Diamine, insbesondere m-Xylylendiamin (MXDA) und cycloaliphatische Diamine, insbesondere Isophorondiamin, N-Cyclohexylpropylen-1,3-diamin und 4,4'-Diaminodicyclohexylmethan (Dicykan). Bevorzugt sind auch difunktionelle oder trifunktionelle primäre Polyetheramine auf der Basis von Polypropylenglykol, wie z. B. Jeffamine® D 230 oder Jeffamine® T 403. Bevorzugt sind auch Mischungen der als bevorzugt genannten Amine, beispielsweise Mischungen, die 2,2-Dimethylpropylenamin und Isophoronamin enthalten.

Zu den alkoholischen Härtern zählen vor allem niedermolekulare und höhermolekulare aliphatische und cycloaliphatische Alkohole. Alkoholische Härter vernetzen Carbonatpolymere durch Reaktion der primären oder sekundären Alkoholfunktionen mit den 1,3-Dioxolan-2-on-Gruppen der Carbonatpolymere unter Ausbildung von Diestern der Kohlensäure. Bevorzugte alkoholische Härter weisen im Mittel wenigstens zwei primäre oder sekundäre Hydroxygruppen pro Molekül, z. B. zwei, drei oder vier primäre oder sekundäre Hydroxygruppen pro Molekül, auf. Geeignete niedermolekulare alkoholische Härter sind z. B. 1,4-Butandiol, Ethylenglykol, Diethylenglykol, Triethylenglykol, Neopentylglykol, 1,3-Propandiol, 1,5-Pentandiol, 1,6-Hexandiol, Glycerin, Diglycerin, Pentaerythrit, Dipentaerythrit, Zuckeralkohole wie Sorbit und Mannit.

Geeignete alkoholische Härter sind auch höhermolekulare, polymere Polyole, beispielsweise Polyesterpolyole, Polycarbonatpolyole, Polyetherpolyole, Polyacrylatpolyole und Polyvinylalkohole.

Geeignete polymere Polyolhärter weisen in der Regel eine mittlere OH-Funktionalität von wenigstens 1,5 mol und speziell wenigstens 1,8, z. B. im Bereich von 1,5 bis 10 und insbesondere im Bereich von 1,8 bis 4, auf. Unter der mittleren OH-Funktionalität versteht man die mittlere Anzahl OH-Gruppen je Polymerkette. Typische polymere Polyolkomponenten weisen in der Regel ein zahlenmittleres Molekulargewicht von etwa 250 bis 50000 g/mol, vorzugsweise von etwa 500 bis 10000 g/mol auf. Vorzugsweise sind wenigstens 50 mol-% der in der polymeren Polyolkomponente enthaltenen Hydroxylgruppen primäre Hydroxylgruppen.

In der Regel handelt es sich bei Polyesterpolyolen um lineare oder verzweigte polymere Verbindungen mit Estergruppen im Polymerrückgrat, die an den Enden der Polymerkette freie Hydroxylgruppen aufweisen. In der Regel handelt es sich hierbei um Polyester, die durch Polykondensation von zweiwertigen Alkoholen mit zweiwertigen Carbonsäuren, gegebenenfalls in Gegenwart höherwertiger Alkohole (z. B. 3, 4, 5 oder 6-wertiger Alkohole) und/oder höherwertiger Polycarbonsäuren, erhalten werden. Anstelle der freien Di- bzw. Polycarbonsäuren können auch die entsprechenden Di- bzw. Polycarbonsäureanhydride oder entsprechende Di- bzw. Polycarbonsäureester von niederen Alkoholen oder deren Gemische zur Herstellung der Polyesterpolyole verwendet werden. Die Di- bzw. Polycarbonsäuren können aliphatisch, cycloaliphatisch, araliphatisch, aromatisch oder heterocyclisch sein, weisen in der Regel 2 bis 50 und insbesondere 4 bis 20 C-Atome auf und können gegebenenfalls, z. B. durch Halogenatome, substituiert und/oder ungesättigt sein. Als Beispiele hierfür seien genannt: Korksäure, Azelainsäure, Phthalsäure, Isophthalsäure, Phthalsäureanhydrid, Tetrahydrophthalsäureanhydrid, Hexahydrophthalsäureanhydrid, Tetrachlorphthalsäureanhydrid, Endomethylentetrahydrophthalsäureanhydrid, Glutarsäureanhydrid, Maleinsäure, Maleinsäureanhydrid, Alkenylbernsteinsäure, Fumarsäure und dimere Fettsäuren. Für die Herstellung der Polyesterpolyole kommen als Diole insbesondere aliphatische und cycloaliphatische Diole mit in der Regel 2 bis 40 und insbesondere 2 bis 20 C-Atomen in Betracht, z. B. Ethylenglykol, Propan-1,2-diol, Propan-1,3-diol, Butan-1,3-diol, Butan-1,4-diol, Buten-1,4-diol, Butin-1,4-diol, Pentan-1,5-diol, Neopentylglykol, Bis-(hydroxymethyl)cyclohexane wie 1,4-Bis-(hydroxymethyl)cyclohexan, 2-Methylpropan-1,3-diol, Methylpentandiole, ferner Diethylenglykol, Triethylenglykol, Tetraethylenglykol, Polyethylenglykol, Dipropylenglykol, Polypropylenglykol, Dibutylenglykol und Polybutylenglykole. Bevorzugt sind Alkohole der allgemeinen Formel HO-(CH₂)ₓ-OH, wobei x eine Zahl von 2 bis 20, bevorzugt eine gerade Zahl von 2 bis 12 ist. Beispiele hierfür sind Ethylenglykol, Butan-1,4-diol, Hexan-1,6-diol, Octan-1,8-diol und Dodecan-1,12-diol. Weiterhin bevorzugt sind Neopentylglykol und Pentan-1,5-diol.

Geeignet als alkoholische Härter sind auch Polyesterpolyole auf Lacton-Basis, wobei es sich um Homo- oder Mischpolymerisate von Lactonen, bevorzugt um endständige Hydroxylgruppen-aufweisende Anlagerungsprodukte von Lactonen an geeignete difunktionelle Startermoleküle handelt. Als Lactone kommen bevorzugt solche in Betracht, die sich von Verbindungen der allgemeinen Formel HO-(CH₂)_{z}-COOH ableiten, wobei z eine Zahl von 1 bis 20 ist und ein H-Atom einer Methyleneinheit auch durch einen C₁- bis C₄-Alkylrest substituiert sein kann. Beispiele sind ε-Caprolacton, β-Propiolacton, γ-Butyrolacton und/oder Methyl-ε-caprolacton sowie deren Gemische. Geeignete Startermoleküle sind z. B. die vorstehend als Aufbaukomponente für die Polyesterpolyole genannten niedermolekularen zweiwertigen Alkohole. Die entsprechenden Polymerisate des ε-Caprolactons sind besonders bevorzugt. Auch niedere Polyesterdiole oder Polyetherdiole können als Starter zur Herstellung der Lacton-Polymerisate eingesetzt sein. Anstelle der Polymerisate von Lactonen können auch die entsprechenden, chemisch äquivalenten Polykondensate der den Lactonen entsprechenden Hydroxycarbonsäuren eingesetzt werden.

Beispiele für geeignete Polyesterpolyole sind z. B. die aus Ullmanns Enzyklopädie der Technischen Chemie, 4. Auflage, Band 19, Seiten 62 bis 65 bekannten Polyesterpolyole.

Ferner kommen auch Polycarbonatpolyole, wie sie z. B. durch Umsetzung von Phosgen mit einem Überschuss von den als Aufbaukomponenten für die Polyesterpolyole genannten niedermolekularen Alkohole erhalten werden können, in Betracht.

Bei den Polyetherpolyolen handelt es sich insbesondere um Polyetherpolyole, die durch Polymerisation von Ethylenoxid, Propylenoxid, Butylenoxid, Tetrahydrofuran, Styroloxid oder Epichlorhydrin mit sich selbst, z. B. in Gegenwart von BF₃ oder durch Anlagerung dieser Verbindungen gegebenenfalls im Gemisch oder nacheinander, an bi- oder polyfunktionelle Startkomponenten mit reaktionsfähigen Wasserstoffatomen, wie Polyole oder polyfunktionelle Amine, z. B. Wasser, Ethylenglykol, Propan-1,2-diol, Propan-1,3-diol, 1,1-Bis-(4-hydroxyphenyl)propan, Trimethylolpropan, Glycerin, Sorbit, Ethanolamin oder Ethylendiamin, hergestellt werden können. Auch Succrosepolyether (siehe DE 1176358 und DE 1064938) sowie auf Formit oder Formose gestartete Polyether (siehe DE 2639083 und DE 2737951) kommen in Frage.

Geeignet sind ebenfalls Polyhydroxyolefine, bevorzugt solche mit 2 endständigen Hydroxylgruppen, z. B. α-ω-Dihydroxypolybutadien.

Geeignet sind ebenfalls Polyhydroxypolyacrylate, wobei die Hydroxylgruppen seitenständig oder endständig angeordnet sein können. Beispiel hierfür sind α,ω-Dihydroxypoly(meth)acrylester, die durch Homo- oder Copolymerisation von Alkylestern der Acrylsäure und/oder der Methacrylsäure in Gegenwart von OH-Gruppen-enthaltenden Reglern wie Mercaptoethanol oder Mercaptopropanol und anschließende Umesterung mit einem niedermolekularen Polyol, beispielsweise einem Alkylenglykol wie Butandiol, erhältlich sind. Solche Polymere sind beispielsweise aus der EP-A 622 378 bekannt. Beispiel hierfür sind weiterhin Polymere, die durch Copolymerisation von Alkylestern der Acrylsäure und/oder der Methacrylsäure mit Hydroxyalkylestern ethylenisch ungesättigter Carbonsäure wie Hydroxyethylacrylat, Hydroxypropylacrylat, Hydroxybutylacrylat, Hydroxyethylmethacrylat, Hydroxypropylmethacrylat oder Hydroxybutylmethacrylat erhältlich sind.

Geeignet sind auch Polyvinylalkohole, die in der Regel durch vollständige oder teilweise Verseifung von Polyvinylestern, insbesondere Polyvinylacetat, erhalten werden können. Sofern die Polyvinylester, bevorzugt Polyvinylacetat, teilverseift vorliegen, liegen bevorzugt höchstens 50 bis 95 % der Estergruppen verseift als Hydroxylgruppen vor. Sofern die Polyvinylester, bevorzugt Polyvinylacetat, vollständig verseift vorliegen, liegen im Allgemeinen mehr als 95 % bis zu 100 % der Estergruppen verseift als Hydroxylgruppen vor.

Unter den höhermolekularen, polymeren Polyolen bevorzugte alkoholische Härter sind insbesondere Polyacrylatpolyole, diese sind beispielsweise unter dem Markennamen Joncryl® der Fa. BASF SE erhältlich, z. B. Joncryl® 945.

Geeignete Härter sind auch Aminosäuren, z. B. Lysin, Arginin, Glutamin und Asparagin und deren Stereoisomere und deren Mischungen.

Selbstredend können auch Mischungen unterschiedlicher Härter eingesetzt werden, z. B. Mischungen eines oder mehrerer aminischer Härter mit einem oder mehreren alkoholischen Härtern, Mischungen eines oder mehrerer aminischer Härter mit einer oder mehreren Aminosäuren oder Mischungen eines oder mehrerer alkoholischer Härter mit einer oder mehreren Aminosäuren.

In den erfindungsgemäßen Bindemittelzusammensetzungen beträgt die Gesamtmenge an Härtern in der Regel 0,1 Gew.-% bis 50 Gew.-%, häufig 0,5 bis 40 Gew.-% und insbesondere 1 bis 30 Gew.-%, bezogen auf die Gesamtmenge an Carbonatpolymeren plus eingesetzten Härtern.

Die Härtung der Bindemittelzusammensetzung kann thermisch durch Erwärmen des Gemischs aus erfindungsgemäßem Polymer und Härter auf eine Temperatur oberhalb der Mischtemperatur erfolgen. Die Härtung kann auch bei niedrigeren Temperaturen erfolgen. Typischerweise erfolgt die Härtung der erfindungsgemäßen Bindemittelzusammensetzungen bei Temperaturen im Bereich von 0 bis 200 °C, vorzugsweise im Bereich von 5 bis 180 °C und insbesondere im Bereich von 10 bis 150 °C. Welche Temperatur geeignet ist, hängt von den jeweiligen Härtern und der gewünschten Härtungsgeschwindigkeit ab und kann im Einzelfall vom Fachmann beispielsweise anhand einfacher Vorversuche ermittelt werden. Im unteren Temperaturbereich (5 bis ca. 35 °C), der ja der meist vorherrschenden Umgebungstemperatur entspricht, reicht es selbstverständlich aus, erfindungsgemäßes Polymer und Härter zu mischen. Alternativ erfolgt die Härtung vorzugsweise mikrowelleninduziert.

Neben den vorgenannten Bestandteilen kann die 2K-Bindemittelzusammensetzung die hierfür üblichen Füllstoffe und/oder Additive enthalten.

Geeignete Füllstoffe sind beispielsweise anorganische oder organische partikelförmige Materialien wie beispielsweise Calciumcarbonate und Silikate sowie anorganische Fasermaterialien wie beispielsweise Glasfaser. Auch organische Füllstoffe wie Kohlefaser und Mischungen aus organischen und anorganischen Füllstoffen, wie beispielsweise Mischungen aus Glas- und Kohlefasern oder Mischungen aus Kohlefasern und anorganischen Füllstoffen, können Anwendung finden. Die Füllstoffe können in einer Menge von 1 bis 75 Gew.-%, bezogen auf das Gesamtgewicht der Zusammensetzung, zugesetzt werden.

Geeignete herkömmliche Additive umfassen beispielsweise Antioxidantien, UV-Absorber/Lichtstabilisatoren, Metalldeaktivatoren, Antistatika, Verstärkungsstoffe, Füllstoffe, Antifoggingmittel, Treibmittel, Biozide, Weichmacher, Gleitmittel, Emulgatoren, Farbmittel, Pigmente, Rheologiemittel, Schlagzähigkeitsverbesserer, Katalysatoren, Adhäsionsregulatoren, optische Aufheller, Flammschutzmittel, Antitropfmittel, Nukleierungsmittel, Lösungsmittel und Reaktivverdünner sowie Gemische davon.

Die gegebenenfalls verwendeten Lichtstabilisatoren/UV-Absorber, Antioxidantien und Metalldeaktivatoren weisen vorzugsweise eine hohe Migrationsstabilität und Temperaturbeständigkeit auf. Sie sind beispielsweise aus den Gruppen a) bis t) ausgewählt. Die Verbindungen der Gruppen a) bis g) und i) stellen Lichtstabilisatoren/UV-Absorber dar, während Verbindungen j) bis t) als Stabilisatoren wirken.
a) 4,4-Diarylbutadiene,
b) Zimtsäureester,
c) Benzotriazole,
d) Hydroxybenzophenone,
e) Diphenylcyanacrylate,
f) Oxamide,
g) 2-Phenyl-1,3,5-triazine,
h) Antioxidantien,
i) Nickelverbindungen,
j) sterisch gehinderte Amine,
k) Metalldesaktivatoren,
l) Phosphite und Phosphonite,
m) Hydroxylamine,
n) Nitrone,
o) Aminoxide,
p) Benzofuranone und Indolinone,
q) Thiosynergisten,
r) Peroxid-zerstörende Verbindungen,
s) Polyamidstabilisatoren und
t) basische Costabilisatoren.

Die Wahl geeigneter herkömmlicher Additive für die erfindungsgemäße Zusammensetzung hängt vom jeweiligen Verwendungszweck der 2K-Bindemittelzusammensetzung ab und kann im Einzelfall vom Fachmann bestimmt werden.

Die erfindungsgemäßen 2K-Bindemittelzusammensetzungen eignen sich insbesondere zur Herstellung von Beschichtungen.

Die Applikation der Bindemittelzusammensetzung zum Zwecke der Herstellung einer Lackschicht kann durch alle üblichen Applikationsmethoden wie z. B. Spritzen, Rakeln, Streichen, Gießen, Tauchen oder Walzen erfolgen. Vorzugsweise werden Spritzapplikationsmethoden angewandt wie zum Beispiel Druckluftspritzen, Airless Spritzen, Hochrotation, elektrostatischer Sprühauftrag (ESTA), gegebenenfalls verbunden mit Heißspritzapplikation wie zum Beispiel Hot-Air-Heißspritzen. Die Applikation kann bei Temperaturen von max. 70 bis 80 °C durchgeführt werden, so dass geeignete Applikationsviskositäten erreicht werden, ohne dass bei der kurzzeitig einwirkenden thermischen Belastung eine Veränderung oder Schädigungen des Beschichtungsmittels und seines gegebenenfalls wiederaufzubereitenden Overspray eintreten. So kann das Heißspritzen so ausgestaltet sein, dass das Beschichtungsmittel nur sehr kurz in der oder kurz vor der Spritzdüse erhitzt wird.

Die für die Applikation verwendete Spritzkabine kann beispielsweise mit einem gegebenenfalls temperierbaren Umlauf betrieben werden, der mit einem geeigneten Absorptionsmedium für den Overspray, z. B. dem Beschichtungsmittel selbst, betrieben wird.

Die Applikation der Bindemittelzusammensetzung kann auch dergestalt durchgeführt werden, dass man die Komponenten erst kurz vor der Applikation in einer Mischkammer vor der Spritzdüse vermischt, wobei diese Applikationsart insbesondere für 2K-Zusammensetzungen mit kurzen Topfzeiten geeignet ist.

Selbstverständlich können die vorstehend beschriebenen Applikationsmethoden auch bei der Herstellung weiterer Lackschichten oder der Basislackschicht im Rahmen der Herstellung eines Mehrschichtaufbaus angewandt werden. Hierbei können jeweils unterschiedliche Beschichtungsstoffe für den Aufbau der verschiedenen Schichten angewandt werden. Bevorzugt ist die Applikation auf einer Basislackschicht.

Als Substrate kommen alle zu lackierenden Oberflächen, die einer kombinierten Härtung zugänglich sind, sowohl grundiert als auch ungrundiert, in Betracht, das sind z. B.: Metalle, Kunststoffe, Holz, Keramik, Stein, Textil, Faserverbunde, Leder, Glas, Glasfasern, Glas- und Steinwolle, mineral- und harzgebundene Baustoffe wie Gips- und Zementplatten oder Dachziegel.

Die erfindungsgemäßen 2K-Bindemittelzusammensetzungen eignen sich insbesondere auch für Klebstoffe.

Als Klebstoffe seien insbesondere 2K-Strukturklebstoffe erwähnt. Strukturklebstoffe dienen zur dauerhaften Verbindung von Formteilen miteinander. Die Formteile können aus beliebigem Material sein; in Betracht kommen Materialien aus Kunststoff, Metall, Holz, Leder, Keramik etc. Es kann sich auch um Fußbodenklebstoffe handeln. Die Zusammensetzungen eignen sich auch als Klebstoffe für die Herstellung von Leiterplatten (electronic circuits), insbesondere auch nach der SMT-Methode (surface mounted technology).

Die folgenden Beispiele dienen der Erläuterung der Erfindung.

### Herstellungsbeispiele:

### Herstellungsbeispiel 1: 5-Methyl-hex-3-in-1,5-diol

Die Synthese erfolgte analog Bull. Acad. Sci. USSR 1965, 683.

In einem 8-L-Reaktor mit 3-stufigem 2-blättrigem, versetztem Scherblattrührer und Thermostat wurden bei 20 °C unter N₂-Atmosphäre 100,0 g (1,384 mol) 3-Butin-1-ol (Reinheit 97,0 %, Fa. Acros) in 3,92 L Toluol (Reinheit 99,9 %, Fa. BASF SE) gelöst und unter Rühren 320,0 g (4,848 mol) KOH (Reinheit 85,0 %, Fa. BASF SE) hinzugegeben. Innerhalb von 20 min wurde eine Mischung von 441,0 mL (6,00 mol) Aceton und 320,9 mL Toluol hinzugegeben. Zu dem Reaktionsansatz wurden langsam 3 L vollentsalztes Wasser gegeben, um den Feststoff vollständig zu lösen. Die Phasen wurden getrennt und die wässrige Phase wurde zweimal mit je 2 L Ethylacetat extrahiert. Das Lösungsmittel der vereinigten organischen Phasen wurde im Vakuum (50 °C, ca. 5 mbar) entfernt. Man erhielt 183,5 g des Produktes.

Die Identität des Produkts mit der Titelverbindung wurde gaschromatographisch überprüft (GC-Methode: ESMA6F, 30 m RTX-5-Amin 1 µm.32mm/80-0-R: 15 °C/min-250).

### Herstellungsbeispiel 2: Essigsäure-5-hydroxy-5-methyl-hex-3-inylester

100 g (0,78 mol) 5-Methyl-hex-3-in-1,5-diol wurden in 800 mL Dichlormethan gelöst und auf 0 °C gekühlt. 113 mL (1,11 mol) Essigsäureanhydrid wurden in einer Portion hinzugegeben. 127 mL (1,25 mol) Triethylamin wurden auf 0-2 °C gekühlt und innerhalb von 20 min hinzugegeben. Die Reaktionsmischung wurde 2 h bei 0 °C gerührt. Die Kühlung wurde entfernt und der Reaktionsansatz wurde 16 h bei 20°C gerührt. Die Mischung wurde auf 0 °C gekühlt und 1200 mL einer 5%igen Salzsäurelösung hinzugegeben, wobei die Temperatur der Reaktionsmischung unter 5 °C gehalten wurde. Der Ansatz wurde dreimal mit je 150 mL tert.-Butylmethylether (MTBE) extrahiert und die vereinigten organischen Phasen wurden viermal je ca. 1 h mit je 400 mL 5%iger wässriger Natriumhydrogencarbonatlösung gerührt, bis jeweils keine Gasentwicklung mehr zu beobachten war. Die organische Phase wurde mit 1 L vollentsalztem Wasser gewaschen, über Natriumsulfat getrocknet und das Lösungsmittel entfernt. Man erhielt 122,21 g (Ausbeute 92 %) einer klaren dunkelgelben Flüssigkeit. Die Reinheit wurde gaschromatographisch zu 99,5 % bestimmt.
¹H-NMR (CDCls, 500 MHz): 1,5 (s, 6H, C(CH₃)₂), 2,1 (s, 3H, C(O)CH₃), 2,5 (t, 2H, CH₂CH₂O), 3,4 (bs, 1 H, OH), 4.1 (t, 2H, CH₂CH₂O) ppm.

### Herstellungsbeispiel 3: 4,4-Dimethyl-5-(3-acetoxypropyliden)-1,3-dioxolan-2-on (exo-VC-OAc)

In einem 300-mL-Autoklaven wurden 50 g Essigsäure-5-hydroxy-5-methyl-hex-3-inylester in 74 mL Toluol vorgelegt. Hierzu gab man 0,9 g Silberacetat und 7,8 g 1,8-Diazabicyclo[5.4.0]undec-7-en (DBU). Der Reaktionsansatz wurde auf 70 °C erwärmt und ein CO₂-Druck von 50 bar wurde eingestellt. Nach 40 h entspannte man auf Normaldruck und wusch den Reaktionsansatz mit zweimal je 100 mL Wasser und mit 100 mL 5%iger Salzsäurelösung. Die vereinigten wässrigen Phasen wurden mit 100 mL Toluol extrahiert, und die vereinigten organischen Phasen wurden über Natriumcarbonat getrocknet. Das Lösungsmittel wurde entfernt, und der erhaltene Rückstand wurde aus 200 g Cyclohexan umkristallisiert. Man erhielt 35 g des Produktes der Titelverbindung (Reinheit > 99 %). Die Identität der Titelverbindung wurde gaschromatographisch überprüft (GC-Methode: 30m FFAP ID = 0,32 mm, FD = 0,25µm; 80°C 6K/min bis 250 °C Temp. Halten; Retentionszeit: 20,6min).
¹H-NMR (CDCls, 500 MHz): 1,5 (s, 6H, C(CH₃)2), 2,1 (s, 3H, C(O)CH₃), 2,5 (t, 2H, CH₂CH₂O), 3,4 (bs, 1 H, OH), 4,1 (t, 2H, CH₂CH₂O) ppm.

### Beispiel 1: [(3Z)-3-(5,5-Dimethyl-2-oxo-1,3-dioxolan-4-yliden)propyl]acrylat (exo-VCA)

280 g (1,31 mol) [(3Z)-3-(5,5-Dimethyl-2-oxo-1,3-dioxolan-4-yliden)propyl]acetat (exo-VC-OAc), 1307 g (13,1 mol) Ethylacetat, 0,28 g 4-Methoxyphenol (MeHQ) und 84 g (30 Gew.-%) Novozym^{®} 435 der Fa. Novozymes wurden zusammengegeben. Der Ansatz wurde 24 h bei 40 °C gerührt. Der Ansatz wurde filtriert, mit Aceton nachgewaschen, und das Lösungsmittel wurde bei 40 °C am Rotationsverdampfer entfernt. Man erhielt 276,7 g des Produktes der Titelverbindung mit einer Reinheit von 92,4 % (GC-Analyse).
¹H-NMR (CDCls, 400 MHz): 1,6 (s, 6H), 2,5 (q, 2H), 4,2 (t, 2H), 4,7 (t, 1H), 5,84-5,87 (dd, 1H), 6,09-6,16 (dd, 1H), 6,37-6,42 (dd, 1H) ppm.

### Polymerisationsbeispiele:

### Beispiel 2: Homopolymerisation von exo-VCA in 1-Methoxy-2-propanol

In einem 250-mL-Kolben mit Rührer wurden 106,5 g 1-Methoxy-2-propanol und 30,0 g exo-VCA vorgelegt (Feststoffgehalt: 20 %). Die Mischung wurde im Stickstoffstrom und unter Rühren auf 120 °C erhitzt. Hierzu gab man innerhalb 3 h eine Lösung von 0,3 g tert.-Butylperoxy-2-ethylhexanoat (Fa. Akzo Nobel) in 14,7 g 1-Methoxy-2-propanol. Der Reaktionsansatz wurde 1 h bei 120 °C gerührt.

Nach dem Abkühlen auf 20°C wurde das Polymer ohne vorhergehende Isolierung mittels Gelpermeationschromatographie (GPC) mit dem Lösungsmittel Tetrahydrofuran analysiert (GPC-Analysator der Fa. Waters, Flussrate: 1 mL THF/min, UV-Detektor Lambda-Max 481, 254 nm, Fa. Waters; vier in Reihe geschaltete PL-Gel-Säulen, Säulenmaterial: vernetzte Polystyrol-Divinylbenzol-Matrix, Teilchengröße 5 µm, Porenweiten: 2 x 500 Angström, 1 x 1000 Angström, 1 x 10000 Angström) . Die Analyse ergab ein Polymer mit Mn = 2940 g/mol und Mw = 6680 g/mol, was einer Dispersität von etwa 2,3 entspricht.

Das ¹H-NMR-Spektrum (CDCl₃, 400 MHz) des Polymers weist unter anderem ein Signal mit chemischer Verschiebung von 4,7 ppm auf, welches dem Wasserstoffatom der zu dem 1,3-Dioxolan-2-on-Ring α-ständigen, ungesättigten CH-Gruppe zugeordnet werden kann, sowie ein Signal mit einer chemischen Verschiebung bei etwa 4,1 ppm, welches der CH₂-Gruppe in α-Position zu dem Acrylatrest zugeordnet werden kann (Referenz: Tetramethylsilan). Das Intensitätsverhältnis beider Signale beträgt 0,99 : 2,00. Das ¹H-NMR-Spektrum bestätigt somit, dass die exocyclische Doppelbindung in der 5-Position des 1,3-Dioxolan-2-on-Rings erhalten geblieben ist. Das ¹³C-NMR-Spektrum (CDCl₃, 100 MHz) zeigt das Signal des Kohlenstoffatoms der Carbonylgruppe des 1,3-Dioxolan-2-on-Rings bei etwa 151 ppm, das Signal des olefinischen endocyclischen Kohlenstoffatoms bei etwa 153 ppm und das Signal des Kohlenstoffatoms der CH-Gruppe, die in α-Position zu dem 1,3-Dioxolan-2-on-Ring steht, bei etwa 96 ppm.

### Beispiel 3: Homopolymerisation von exo-VCA in Dimethylcarbonat

In einem 250-mL-Kolben mit Rührer wurden 35,0 g Dimethylcarbonat und 10,0 g exo-VCA vorgelegt (Feststoffgehalt: 17 %). Die Mischung wurde im Stickstoffstrom und unter Rühren auf 85 °C erhitzt. Hierzu gab man innerhalb 3 h eine Lösung von tert.-Butylperoxypivalat (0,3 g, 75 % in Kohlenwasserstoffen, Fa. Pergan) in 14,9 g Dimethylcarbonat. Der Reaktionsansatz wurde 1 h bei 85 °C gerührt.

Das Polymer wurde ohne vorhergehende Isolierung mittels GPC (Lösungsmittel Tetrahydrofuran) analysiert. Die Analyse ergab ein Polymer mit Mn = 1153 g/mol und Mw = 36769 g/mol, was einer Dispersität von etwa 31,9 entspricht. Das ¹H-NMR-Spektrum zeigt die gleichen Signale und Relativintensitäten wie das Spektrum von Beispiel 2.

### Beispiel 4: Homopolymerisation von exo-VCA in Glyme

Die Herstellung erfolgte analog Beispiel 3, wobei das Lösungsmittel Glyme war und der Feststoffgehalt 30 % betrug.

Nach dem Abkühlen auf 20 °C wurde das Polymer ohne vorhergehende Isolierung mittels Gelpermeationschromatographie (GPC) mit dem Lösungsmittel Tetrahydrofuran analysiert. Die Analyse ergab ein Polymer mit Mn = 1123 g/mol und Mw = 35004 g/mol, was einer Dispersität von etwa 31,2 entspricht.

### Beispiel 5: Homopolymerisation von exo-VCA in Anisol

Die Herstellung erfolgte analog Beispiel 3, wobei das Lösungsmittel Anisol war und der Feststoffgehalt 30 % betrug.

Nach dem Abkühlen auf 20 °C wurde das Polymer ohne vorhergehende Isolierung mittels Gelpermeationschromatographie (GPC) mit dem Lösungsmittel Tetrahydrofuran analysiert. Die Analyse ergab ein Polymer mit Mn = 711 g/mol und Mw = 120710 g/mol, was einer Dispersität von etwa 169,8 entspricht.

### Beispiel 6: Copolymerisation von exo-VCA und n-Butylacrylat, 20 : 80 w/w

In einem 250-mL-Kolben mit Rührer wurden 54,0 g Dimethylcarbonat, 2,4 g n-Butylacrylat und 6,0 g exo-VCA vorgelegt (Feststoffgehalt: 25 %). Die Mischung wurde im Stickstoffstrom und unter Rühren auf 85 °C erhitzt. Hierzu gab man innerhalb 3,5 h eine Lösung von 0,4 g tert.-Butylperoxypivalat (75 % in Kohlenwasserstoffen, Fa. Pergan) in 15,0 g Dimethylcarbonat. 15 min nach Beginn der Zugabe wurde eine Lösung von 21,6 g n-Butylacrylat in 21,6 g Dimethylcarbonat über 3 h hinzugegeben. Nachdem beide Lösungen vollständig zugegeben waren, wurde der Reaktionsansatz 1 h bei 85 °C gerührt.

Das ¹H-NMR-Spektrum (CDCl₃, 400 MHz) des Polymers weist unter anderem ein Signal mit chemischer Verschiebung von 4,7 ppm, welches dem Wasserstoffatom der zu dem 1,3-Dioxolan-2-on-Ring α-ständigen, ungesättigten CH-Gruppe zugeordnet werden kann, sowie ein Signal mit einer chemischen Verschiebung bei etwa 4,1 ppm, welches der CH₂-Gruppe in α-Position zu dem Acrylatrest und der CH₂-O-Gruppe des n-Butylacrylats zugeordnet werden kann (Referenz: Tetramethylsilan), auf. Das Intensitätsverhältnis zwischen dem Signal des CH-Protons der ungesättigten Bindung am Carbonatring und der überlappenden Signale der CH₂-O-Gruppe liegt bei 1 : 10, wie bei einem Verhältnis von exo-VCA und n-Butylacrylat von 20 : 80 w/w erwartet.

### Beispiel 7: Copolymerisation von exo-VCA, Styrol und 2-Ethylhexylacrylat (EHA), 35 : 35 : 30 w/w

56,4 g n-Butylacetat wurden in einen 250-mL-Kolben gegeben und im Stickstoffstrom und unter Rühren auf 120 °C erhitzt. Hierzu gab man innerhalb von 2 h gleichzeitig eine Mischung aus 4,8 g tert.-Butylperoctoat und 24,0 g n-Butylacetat und eine Mischung aus 42,0 g Styrol, 36,0 g 2-Ethylhexylacrylat (EHA) und 42,0 g exo-VCA. Nachdem beide Mischungen zugegeben waren, gab man innerhalb von 15 min eine Mischung aus 0,6 g tert.-Butylperoctoat und 3,0 g n-Butylacetat hinzu und rührte den Reaktionsansatz anschließend 2 h bei 120 °C.

Der Feststoffgehalt betrug 38 %. Die GPC-Analyse mit Tetrahydrofuran ergab ein Polymer mit Mn = 2009 g/mol und Mw = 8775 g/mol, was einer Dispersität von etwa 4,3 entspricht.

### Beispiel 8: Copolymerisation von exo-VCA, Styrol und 2-Ethylhexylacrylat (EHA), 35 : 35 : 30 w/w

Die Herstellung erfolgte analog Beispiel 7, wobei insbesondere die Menge des vorgelegten n-Butylacetats variiert wurde.

Der Feststoffgehalt betrug 60,1 %. Die GPC-Analyse mit Tetrahydrofuran ergab ein Polymer mit Mn = 2887 g/mol und Mw = 17818 g/mol, was einer Dispersität von etwa 6,2 entspricht.

### Beispiel 9: Copolymerisation von exo-VCA, Styrol und 2-Ethylhexylacrylat (EHA), 35 : 35 : 30 w/w

Die Herstellung erfolgte analog Beispiel 7, wobei insbesondere die Menge des vorgelegten n-Butylacetats variiert wurde.

Der Feststoffgehalt betrug 72,5 %. Die GPC-Analyse mit Tetrahydrofuran ergab ein Polymer mit Mn = 3285 g/mol und Mw = 26100 g/mol, was einer Dispersität von etwa 8,0 entspricht.

### Beispiel 10: Copolymerisation von exo-VCA, Styrol und 2-Ethylhexylacrylat (EHA), 35 : 35 : 30 w/w

Die Herstellung erfolgte analog Beispiel 7, wobei insbesondere die Menge des vorgelegten n-Butylacetats variiert wurde.

Der Feststoffgehalt betrug 80,2 %. Die GPC-Analyse mit Tetrahydrofuran ergab ein Polymer mit Mn = 4565 g/mol und Mw = 58671 g/mol, was einer Dispersität von etwa 12,9 entspricht.

### Beispiel 11: Copolymerisation von exo-VCA, Styrol und 2-Ethylhexylacrylat (EHA), 35 : 35 : 30 w/w

47,0 g 1-Methoxy-2-methyl-2-propanol wurden in einen 250-mL-Kolben gegeben und im Stickstoffstrom und unter Rühren auf 120 °C erhitzt. Hierzu gab man innerhalb von 2 h gleichzeitig eine Mischung aus 8,0 g tert.-Butylperoctoat und 40,0 g 1-Methoxy-2-methyl-2-propanol und eine Mischung aus 42,0 g Styrol, 36,0 g 2-Ethylhexylacrylat (EHA) und 42,0 g exo-VCA. Nachdem beide Mischungen zugegeben waren, gab man innerhalb von 15 min eine Mischung aus 0,5 g tert.-Butylperoctoat und 2,5 g 1-Methoxy-2-methyl-2-propanol hinzu und rührte den Reaktionsansatz anschließend 2 h bei 120 °C.

Der Feststoffgehalt betrug 61,0 %. Die GPC-Analyse mit Tetrahydrofuran ergab ein Polymer mit Mn = 3249 g/mol und Mw = 15192 g/mol, was einer Dispersität von etwa 4,7 entspricht.

### Beispiel 12: Copolymerisation von exo-VCA, Styrol und 2-Ethylhexylacrylat (EHA), 35 : 35 : 30 w/w

94,0 g n-Butylacetat, 7,0 g Styrol und 7,0 g exo-VCA wurden in einen 250-mL-Kolben gegeben und im Stickstoffstrom und unter Rühren auf 120 °C erhitzt. Hierzu gab man innerhalb von 2 h gleichzeitig eine Mischung aus 13,6 g tert.-Butylperoctoat und 68,0 g n-Butylacetat und eine Mischung aus 63,0 g Styrol, 69,0 g 2-Ethylhexylacrylat (EHA) und 72,4 g exo-VCA. Nachdem beide Mischungen zugegeben waren, gab man innerhalb von 15 min eine Mischung aus 1,0 g tert.-Butylperoctoat und 5,0 g n-Butylacetat hinzu und rührte den Reaktionsansatz anschließend 2 h bei 120 °C.

Der Feststoffgehalt betrug 60,3 %. Die GPC-Analyse mit Tetrahydrofuran ergab ein Polymer mit Mn = 2546 g/mol und Mw = 15298 g/mol, was einer Dispersität von etwa 6,0 entspricht.

### Beispiel 13: Copolymerisation von exo-VCA und 2-Ethylhexylacrylat (EHA), 50 : 50 w/w

72,0 g n-Butylacetat, 3,6 g 2-Ethylhexylacrylat (EHA) und 18,0 g exo-VCA wurden in einen 250-mL-Kolben gegeben und im Stickstoffstrom und unter Rühren auf 85 °C erhitzt. Hierzu gab man innerhalb von 3,5 h eine Mischung aus 0,48 g tert.-Butylperoxypivalat (75 % in Kohlenwasserstoffen, Fa. Pergan) und 18,0 g n-Butylacetat. 15 min nach Beginn der Zugabe gab man eine Mischung aus 14,4 g 2-Ethylhexylacrylat (EHA) und 14,4 g n-Butylacetat über 3 h hinzu. Nachdem beide Mischungen zugegeben waren, rührte man den Reaktionsansatz 1 h bei 85 °C. Anschließend gab man innerhalb von 15 min eine Mischung aus 0,48 g tert.-Butylperoxypivalat (75 % in Kohlenwasserstoffen, Fa. Pergan) und 18,0 g n-Butylacetat innerhalb von 5 min hinzu und rührte den Reaktionsansatz anschließend 1 h bei 85 °C.

Der Feststoffgehalt betrug 22,9 %. Die GPC-Analyse mit Tetrahydrofuran ergab ein Polymer mit Mn = 4591 g/mol und Mw = 42040 g/mol, was einer Dispersität von etwa 9,2 entspricht.

### Beispiel 14: Copolymerisation von exo-VCA und 2-Ethylhexylacrylat (EHA), 50 : 50 w/w

105,0 g n-Butylacetat wurden in einen 250-mL-Kolben gegeben und im Stickstoffstrom und unter Rühren auf 120 °C erhitzt. Hierzu gab man innerhalb von 2 h gleichzeitig eine Mischung aus 2,8 g tert.-Butylperoctoat und 28,0 g n-Butylacetat und eine Mischung aus 35,0 g 2-Ethylhexylacrylat (EHA), 35,0 g exo-VCA und 35,0 g n-Butylacetat. Nachdem beide Mischungen zugegeben waren, gab man innerhalb von 15 min eine Mischung aus 0,35 g tert.-Butylperoctoat und 3,5 g n-Butylacetat hinzu und rührte den Reaktionsansatz anschließend 2 h bei 120 °C.

Der Feststoffgehalt betrug 30,1 %. Die GPC-Analyse mit Tetrahydrofuran ergab ein Polymer mit Mn = 894 g/mol und Mw = 2743 g/mol, was einer Dispersität von etwa 3,1 entspricht.

### Beispiel 15: Copolymerisation von exo-VCA und Methylacrylat, 70 : 30 w/w

250,0 g 1-Methoxy-2-methyl-2-propanol wurden in einen 1-L-Glasreaktor gegeben und im Stickstoffstrom und unter Rühren auf 120 °C erhitzt. Hierzu gab man innerhalb von 2 h gleichzeitig eine Mischung aus 22,4 g tert.-Butylperoctoat und 89,6 g 1-Methoxy-2-methyl-2-propanol und eine Mischung aus 126,0 g Methylacrylat, 294,0 g exo-VCA und 100,8 g 1-Methoxy-2-methyl-2-propanol. Nachdem beide Mischungen zugegeben waren, gab man innerhalb von 15 min eine Mischung aus 2,0 g tert.-Butylperoctoat und 8,0 g 1-Methoxy-2-methyl-2-propanol hinzu und rührte den Reaktionsansatz anschließend 2 h bei 120 °C. Nach Abkühlen auf Raumtemperatur wurde die untere, polymerangereicherte Phase (656,7 g, 55,2 %) am Rotationsverdampfer auf 443,5 g (81,7 %) aufkonzentriert. Anschließend wurde der Reaktionsansatz mit 74,2 g Dimethylcarbonat verdünnt, wobei die Reaktionsmischung wieder klar wurde und die Viskosität leicht abnahm.

Der Feststoffgehalt betrug 50 %. Die GPC-Analyse mit Tetrahydrofuran ergab ein Polymer mit Mn = 1299 g/mol und Mw = 9983 g/mol, was einer Dispersität von etwa 7,7 entspricht.

Anwendungsbeispiele:
a) Vernetzung des Homopolymers aus Beispiel 2 und Untersuchung der mechanischen Eigenschaften sowie Lösungsmittelbeständigkeit des Polymerfilms.

### Beispiel 16: Vernetzung bei Zimmertemperatur, ca. 20 % Feststoffgehalt

Zu 2 mL einer Lösung des Homopolymers aus Beispiel 2 in 1-Methoxy-2-propanol mit einen Feststoffgehalt von ca. 20 % gab man 0,12 g reines m-Xylendiamin (MXDA) als Härter hinzu und trug die Mischung bei Zimmertemperatur innerhalb weniger Minuten auf ein phosphatiertes Stahlblech auf. Nach 20 min war der Film trocken. Der Film wurde nach 24 h auf seine mechanischen Eigenschaften sowie seine Lösungsmittelbeständigkeit überprüft (siehe Tabelle 1).

### Beispiel 17: Vernetzung bei 100 °C, ca. 20 % Feststoffgehalt

Zu 2 mL einer Lösung des Homopolymers aus Beispiel 2 in 1-Methoxy-2-propanol mit einen Feststoffgehalt von ca. 20 % gab man 0,12 g reines m-Xylendiamin (MXDA) als Härter hinzu, härtete für 10 min bei 100 °C und trug die Mischung auf ein phosphatiertes Stahlblech auf. Nach 20 min war der Film trocken. Der Film wurde nach 24 h auf seine mechanischen Eigenschaften sowie seine Lösungsmittelbeständigkeit überprüft (siehe Tabelle 1).

### Beispiel 18: Vernetzung bei Zimmertemperatur, ca. 80 % Feststoffgehalt

Das Homopolymer aus Beispiel 2 wurde in einer Menge eingesetzt, so dass der Feststoffgehalt der Lösung ca. 80 % betrug. Der Härter MXDA wurde in gleichem stöchiometrischem Verhältnis wie in den Beispielen 16 und 17 hinzugegeben und das Polymer bei Zimmertemperatur binnen weniger Minuten auf ein phosphatiertes Stahlblech aufgetragen. Der Film wurde nach 24 h auf seine mechanischen Eigenschaften sowie seine Lösungsmittelbeständigkeit überprüft (siehe Tabelle 1).

Die Bestimmung der Pendeldämpfung (Anzahl der Ausschläge) erfolgte in Anlehnung an die Methode von König, beschrieben in DIN EN ISO 1522.

Die Bestimmung der Erichsentiefung erfolgte nach ISO 1520.

Die Bestimmung der Lösungsmittelbeständigkeit erfolgte nach DIN 68861-1.

Der Gitterschnitt erfolgte nach DIN ISO 2409 auf Bonderblech.

**Tabelle 1: Mechanische Untersuchung und Untersuchung der Lösungsmittelbeständigkeit des vernetzten Homopolymerfilms.**

| | Polymerfilm nach Beispiel 16 | Polymerfilm nach Beispiel 17 | Polymerfilm nach Beispiel 18 |
|---|---|---|---|
| Pendeldämpfung | 117 | 127 | 120 |
| Erichsentiefung | 3,8 | 3,5 | 2,9 |
| Beständigkeit gegenüber Ethanol/Wasser | 0 | 0 | 4 |
| Beständigkeit gegenüber Ethylacetat/Ethanol | 0 | 0 | 0 |
| Beständigkeit gegenüber Xylol | 0 | 0 | 0 |

b) Vernetzung erfindungsgemäßer Polymere, gegebenenfalls unter Verwendung unterschiedlicher Mengen unterschiedlicher Katalysatoren, und Untersuchung der mechanischen Eigenschaften sowie Lösungsmittelbeständigkeit der Polymerfilme.

### Vernetzung mit Polyolhärter (allgemeine Versuchsvorschrift):

1,86 g eines analog zu Beispiel 7 hergestellten Copolymers (58,6 Gew.-% in n-Butylacetat), 3,9 g Joncryl® 945 (Fa. BASF SE; Polyacrylatpolyol, OH-Zahl = 180, Glasübergangstemperatur Tg = 17 °C, Feststoffgehalt 76 Gew.-% in n-Butylacetat) und der jeweilige Katalysator wurden zusammengegeben. Man vermengte die Einsatzstoffe, bis eine homogene Mischung entstanden war. Die Mischung wurde zügig mit einem Kastenrakel auf das jeweilige Substrat aufgetragen. Zur Bestimmung der Filmhärte wurde die Mischung mit einem 200-µm-Kastenrakel auf Glas und zur Bestimmung der Elastizität und Lösungsmittelbeständigkeit wurde die Mischung mit einem 150-µm-Kastenrakel auf Bonderblech (Gardobond®) aufgetragen. Der aufgetragene Film wurde 10 min bei Raumtemperatur abgelüftet und anschließend bei 100 °C oder 150 °C für 120 min im Trockenofen gehärtet (Aushärtung). Die Untersuchung der gehärteten Filme erfolgte nach eintägigem Lagern in einem Klimaraum bei 20 °C und 42 % Luftfeuchtigkeit.

| | Beispiel 19 | Beispiel 20 | Beispiel 21 | Beispiel 22 |
|---|---|---|---|---|
| Aushärtung/°C | 100 | 150 | 100 | 150 |
| Katalysator | DBU | DBU | DBU | DBU |
| Katalysatormenge/g | 0,45 | 0,45 | 0,09 | 0,09 |
| Topfzeit/min | 10 | 10 | >240 | >240 |
| Pendeldämpfung | 105 | 128 | 50 | 131 |
| Erichsentiefung | 8,9 | 8,8 | 9,4 | 8,6 |
| Beständigkeit gegenüber Ethylacetat/Ethanol | 1 | 1 | 1 | 5 |
| Gitterschnitt (G/T) | 0-1 | 0 | 3 | 2-3 |
| Aussehen | glatt, klar | glatt, klar, leicht gelb | glatt, klar | glatt, klar, leicht gelb |

| | | | | |
|---|---|---|---|---|
| DBU = 1,8-Diazabicyclo[5.4.0]undec-7-en | | | | |

| | Beispiel 23 | Beispiel 24 | Beispiel 25 | Beispiel 26 |
|---|---|---|---|---|
| Aushärtung/°C | 100 | 150 | 100 | 150 |
| Katalysator | TPP | TPP | TEA | TEA |
| Katalysatormenge/g | 0,9 | 0,9 | 0,9 | 0,9 |
| Topfzeit/min | >240 | >240 | >240 | >240 |
| Pendeldämpfung | 26 | 60 | 24 | 72 |
| Erichsentiefung | 9,5 | 9,5 | 8,5 | 9,4 |
| Beständigkeit gegenüber Ethylacetat/Ethanol | 5 | 5 | 5 | 5 |
| Gitterschnitt (G/T) | 4-5 | 4-5 | 4-5 | 4-5 |
| Aussehen | glatt, klar | glatt, klar, leicht gelb | glatt, klar | glatt, klar, leicht gelb |

| | | | | |
|---|---|---|---|---|
| TPP = Triphenylphosphin TEA = Triethylamin | | | | |

### Vernetzung mit Aminhärter (allgemeine Versuchsvorschrift):

Die angegebene Menge des angegebenen Copolymers und das jeweilige Amin wurden zusammengegeben. Die Zugabe eines Katalysators war nicht notwendig. Man vermengte die Einsatzstoffe, bis eine homogene Mischung entstanden war. Die Mischung wurde zügig mit einem Kastenrakel auf das jeweilige Substrat aufgetragen. Zur Bestimmung der Filmhärte wurde die Mischung mit einem 200-µm-Kastenrakel auf Glas und zur Bestimmung der Elastizität und Lösungsmittelbeständigkeit wurde die Mischung mit einem 150-µm-Kastenrakel auf Bonderblech (Gardobond®) aufgetragen. Der aufgetragene Film wurde 10 min bei Raumtemperatur abgelüftet und anschließend bei 50 °C, 60 °C, 100 °C oder 150 °C für 30 min im Trockenofen gehärtet oder für 24 h bei Raumtemperatur gehärtet (Aushärtung). Die Untersuchung der gehärteten Filme erfolgte nach eintägigem Lagern in einem Klimaraum bei 20 °C und 42 % Luftfeuchtigkeit.

Die Bestimmung der Topfzeiten erfolgte mit dem in Beispiel 7 hergestellten Copolymer (38 Gew.-% in n-Butylacetat).

| | Beispiel 27 | Beispiel 28 | Beispiel 29 | Beispiel 30 |
|---|---|---|---|---|
| Aushärtung/°C | 22 | 22 | 22 | 50 |
| Menge Copolymer aus Beispiel 8/g | 4,798 | 4,765 | 4,872 | 4,798 |
| Amin | MXDA | IPA | Mischung 1 | MXDA |
| Aminmenge/mg (Menge in Bezug auf 1 Mol Carbonatgruppen) | 152 (50 mol-%) | 377 (100 mol-%) | 147 (50 mol-%) | 152 (50 mol-%) |
| Topfzeit/min | 25 | >60 | 5 | 25 |
| Pendeldämpfung | 20 | 14 | 18 | 52 |
| Erichsentiefung | 9,5 | 9,5 | 9,5 | 9,5 |
| Beständigkeit gegenüber Ethylacetat/Ethanol | 3 | 3 | 4 | 3 |
| Gitterschnitt (G/T) | 0 | 0 | 0 | 0 |
| Aussehen | glatt, matt | glatt, matt | glatt, matt | glatt, glänzend |

| | | | | |
|---|---|---|---|---|
| MXDA = m-Xylylendiamin IPA = Isophorondiamin Mischung 1 = Mischung aus 72,1 mg Diethylentriamin, 71,4 mg 2,2-Dimethylpropylendiamin und 119,0 mg Isophorondiamin | | | | |

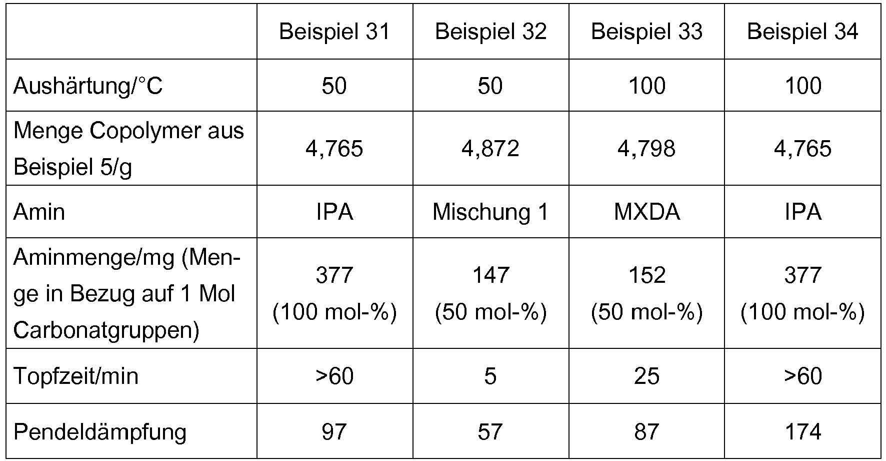

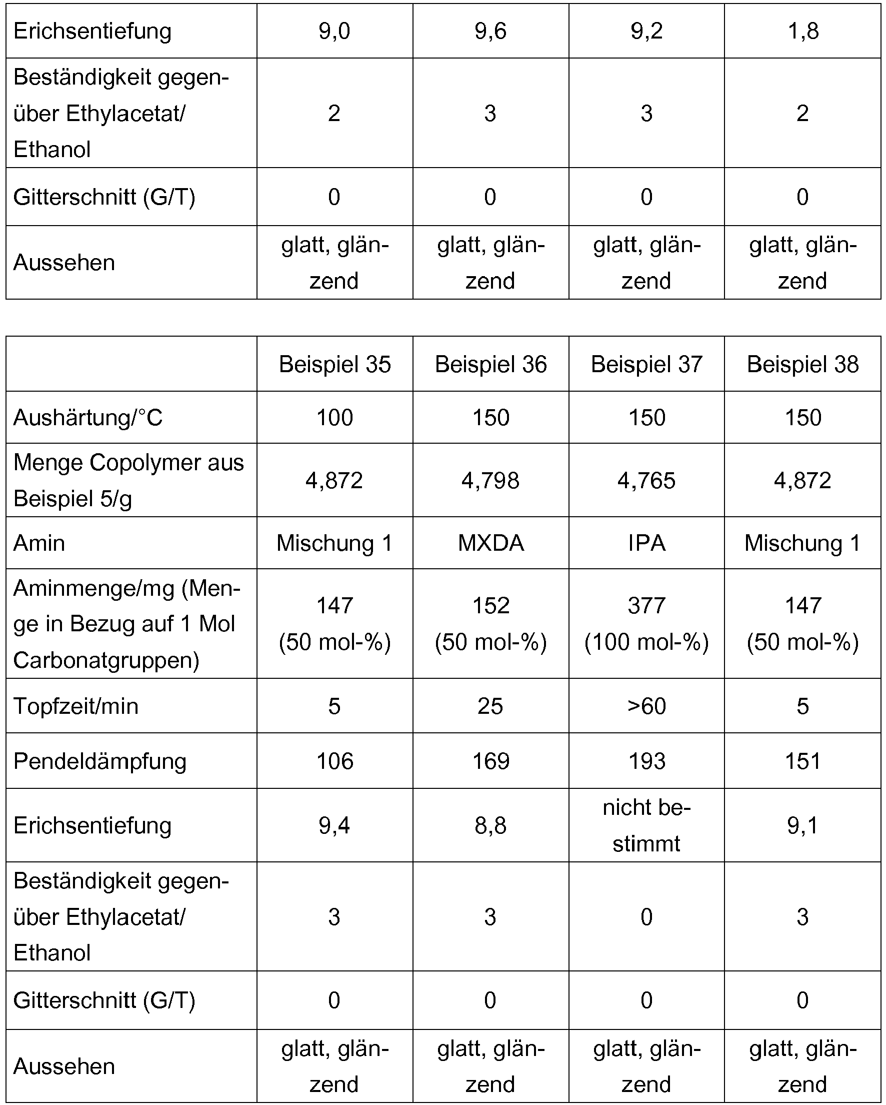

In Beispiel 42 wurde als Härter eine Mischung aus Aminhärter und Polyolhärter verwendet. Die Zugabe eines Katalysators war nicht notwendig.

| | Beispiel 39 | Beispiel 40 | Beispiel 41 | Beispiel 42 |
|---|---|---|---|---|
| Aushärtung/°C | 60 | 50 | 100 | 100 |
| Polymer | Copolymer analog Beispiel 7 | aus Beispiel 4 | exo-VCA Homopolymer analog Beispiel 2, 50 Gew.-% in Tetrahydrofuran | aus Beispiel 8 |

| | | | | |
|---|---|---|---|---|
| Menge Polymer/g | 12,52 | 5,01 | 0,985 | 10,24 |
| polymeres Amin | Jeffamine® D 230 | Jeffamine® T 403 | IPA | IPA |
| Aminmenge/mg (Menge in Bezug auf 1 Mol Carbonatgruppen) | 1305 | 439 (40 mol-%) | 92,7 (50 mol-%) | 830 |
| Polyol | | - | - | Joncryl® 945 |
| Polyolmenge/g | - | - | - | 3,9 |
| Topfzeit/min | >180 | 15 bis 16 | 45 bis 135 | 50 |
| Pendeldämpfung | 63 | 113 | 194 | 167 |
| Erichsentiefung | >9,5 | 8,4 | 0,6 | 6,3 |
| Beständigkeit gegenüber Ethylacetat/Ethanol | nicht bestimmt | 1 | nicht bestimmt | 2 |
| Gitterschnitt (G/T) | 0 | 0 | 0 | 3 |
| Aussehen | glänzend | klar | glatt, klar, einzelne Krater | glatt |

Jeffamine® D 230 ist ein von der Fa. Huntsman kommerziell erhältliches primäres Polyetherdiamin mit einem Molekulargewicht Mw von etwa 230 auf Basis von etwa 2,5 Polypropylenglykol-Wiederholungseinheiten. Jeffamine® T 403 ist ein von der Fa. Huntsman kommerziell erhältliches primäres Polyethertriamin mit einem Molekulargewicht Mw von etwa 440 auf Basis von etwa 5 bis 6 Polypropylenglykol-Wiederholungseinheiten.
c) Vernetzung des Copolymers aus Beispiel 15 (exo-VCA und Methylacrylat, 70 : 30 w/w) mit unterschiedlichen Mengen unterschiedlicher Amine

Die angegebene Menge des in Beispiel 15 hergestellten Copolymers in 60-gewichtsprozentiger Lösung in 1-Methoxy-2-methyl-2-propanol und das jeweilige Amin wurden zusammengegeben. Die Zugabe eines Katalysators war nicht notwendig. Man vermengte die Einsatzstoffe, bis eine homogene Mischung entstanden war. Die Mischung wurde zügig mit einem Kastenrakel auf das jeweilige Substrat aufgetragen. Zur Bestimmung der Filmhärte wurde die Mischung mit einem 200-µm-Kastenrakel auf Glas und zur Bestimmung der Elastizität und Lösungsmittelbeständigkeit wurde die Mischung mit einem 150-µm-Kastenrakel auf Bonderblech (Gardobond®) aufgetragen. Der aufgetragene Film wurde 10 min bei Raumtemperatur abgelüftet und anschließend bei 130 °C für 15 min im Trockenofen gehärtet oder für die angegebene Zeit bei Raumtemperatur gehärtet (Aushärtung). Sofern die Untersuchung der gehärteten Filme nach mehrtägigem Lagern angegeben ist, erfolgte die Lagerung in einem Klimaraum bei 20 °C und 42 % Luftfeuchtigkeit.

| | Beispiel 43 | Beispiel 44 | Beispiel 45 | Beispiel 46 |
|---|---|---|---|---|
| Aushärtung/°C | 130 | 130 | 130 | 130 |
| Menge Copolymer aus Beispiel 15/g | 8,33 | 8,33 | 8,33 | 8,33 |
| Amin | Dicykan | MXDA | Jeffamine® D 230 | Jeffamine® D 230 |
| Aminmenge/g | 1,43 | 0,93 | 3,13 | 1,55 |
| Topfzeit/min | 15 | ca. 4 | 35 | 40 |
| Pendeldämpfung | 176,4 | 190,4 | 172,2 | 204,4 |
| Pendeldämpfung nach 3 Tagen Lagerung | 196 | 204 | 197 | nicht bestimmt |
| Erichsentiefung | 5 | 4,2 | 7,6 | 7,8 |
| Beständigkeit gegenüber Ethylacetat/Ethanol | 0 | 0 | 4 | 4 |
| Beständigkeit gegenüber Toluol | 0 | 0 | 0 | 0 |
| Gitterschnitt (G/T) | 0 | 0 | 0 | 0 |

| | | | | |
|---|---|---|---|---|
| Dicykan = 4,4'-Diaminodicyclohexylmethan | | | | |

| | Beispiel 47 | Beispiel 48 | Beispiel 49 | Beispiel 50 | Beispiel 51 |
|---|---|---|---|---|---|
| Aushärtung/°C | 22 | 22 | 22 | 22 | 22 |
| Menge Copolymer aus Beispiel 15/g | 8,33 | 8,33 | 8,33 | 8,33 | 8,33 |
| Amin | Dicykan | MXDA | MXDA | Jeffamine® D 230 | Jeffamine® D 230 |
| Aminmenge/g | 1,43 | 1,85 | 0,93 | 3,13 | 1,55 |
| Topfzeit/min | 15 | 1 | ca. 4 | 35 | 40 |
| Pendeldämpfung nach 2,5 h | nicht bestimmt | 63 | nicht bestimmt | nicht bestimmt | nicht bestimmt |
| Pendeldämpfung nach 3 Tagen Lagerung | 119 | 133 | 161 | 30 | 146 |
| Pendeldämpfung nach 7 Tagen Lagerung | 134 | 125 | 153 | 24 | 155 |
| Erichsentiefung | 4 | 7,8 | 2,3 | 8,4 | 8,1 |
| Beständigkeit gegenüber Ethylacetat/Ethanol | 0 | 5 | 1 | 5 | 4 |
| Beständigkeit gegenüber Toluol | 0 | 3 | 0 | 3 | 0 |
| Gitterschnitt (G/T) | 0 | 0 | 0 | 0 | 0 |

## Patentansprüche

1. Verbindungen der Formel I, 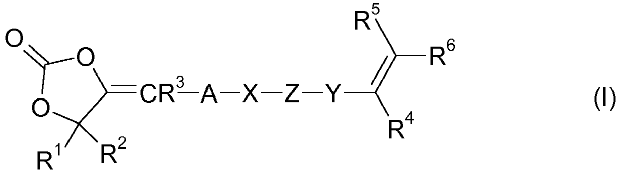 worin
R¹ und R² unabhängig voneinander für Wasserstoff, C₁-C₆-Alkyl, C₁-C₄-Alkoxy-C₁-C₄-alkyl, C₅-C₆-Cycloalkyl, Phenyl oder Phenyl-C₁-C₄-alkyl stehen;
R³ für Wasserstoff, C₁-C₆-Alkyl, C₁-C₄-Alkoxy-C₁-C₄-alkyl, C₅-C₆-Cycloalkyl, Phenyl oder Phenyl-C₁-C₄-alkyl steht;
R⁴ für Wasserstoff, C₁-C₄-Alkyl, CH₂COOR⁸, Phenyl oder Phenyl-C₁-C₄-alkyl steht;
R⁵ und R⁶ unabhängig voneinander für Wasserstoff oder C₁-C₄-Alkyl stehen oder einer der Reste R⁵ oder R⁶ auch für COOR⁸ oder CH₂COOR⁸ stehen kann;
A für eine chemische Bindung oder C₁-C₄-Alkandiyl steht;
X für O oder NR⁷ steht;
Z für eine chemische Bindung, PO₂, SO₂ oder C=O steht;
Y für eine chemische Bindung, CH₂ oder CHCH₃ steht;
R⁷ sofern vorhanden, für C₁-C₆-Alkyl steht;
R⁸ sofern vorhanden, für Wasserstoff oder C₁-C₆-Alkyl steht.

2. Verbindungen nach Anspruch 1, worin R¹ und R² jeweils für Wasserstoff oder C₁-C₆-Alkyl stehen.

3. Verbindungen nach einem der vorhergehenden Ansprüche, worin R³ für Wasserstoff steht.

4. Verbindungen nach einem der vorhergehenden Ansprüche, worin
A für Ethandiyl steht;
X für O steht; und
Z für C=O steht.

5. Verbindungen nach einem der vorhergehenden Ansprüche, worin Y für eine chemische Bindung steht.

6. Verfahren zur Herstellung von Verbindungen der Formel I, wobei eine Verbindung der allgemeinen Formel II 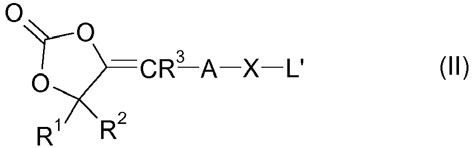 mit einer Verbindung der allgemeinen Formel III 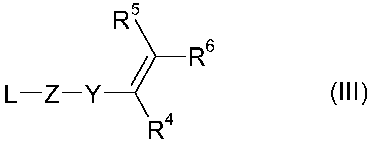 umgesetzt wird, wobei
L für eine nucleophil verdrängbare Abgangsgruppe steht;
L' für Wasserstoff oder eine C₁-C₄-Alkylcarbonylgruppe steht
und A, X, Y, Z, R¹, R², R³, R⁴, R⁵ und R⁶ die in einem der vorhergehenden Ansprüche genannten Bedeutungen aufweisen.

7. Verfahren nach Anspruch 6, wobei L für OH oder C₁-C₈-Alkoxy steht, Z für C=O steht und X für O steht und wobei die Umsetzung unter den Bedingungen einer Veresterung oder Umesterung durchgeführt wird.

8. Polymer, das ein aus Kohlenstoff-Atomen aufgebautes Polymerrückgrat aufweist, an das funktionelle Gruppen der Formel I' angebunden sind, 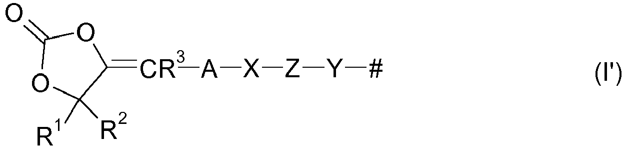 wobei # für die Anbindung an das Polymerrückgrat steht und R¹, R², R³, A, X, Z und Y die in einem der Ansprüche 1 bis 5 genannten Bedeutungen aufweisen.

9. Polymer, das aus polymerisierten ethylenisch ungesättigten Verbindungen M aufgebaut ist, wobei die Verbindungen M wenigstens 1 Gew.-%, bezogen auf die Gesamtmenge der das Polymer bildenden ethylenisch ungesättigten Verbindungen, wenigstens einer Verbindung der Formel I gemäß Anspruch 1 umfassen.

10. Polymer nach einem der Ansprüche 8 oder 9 in Form eines Homopolymers der Verbindung der Formel I.

11. Polymer nach einem der Ansprüche 8 oder 9, das 1 bis 99 Gew.-% wenigstens einer Verbindung der Formel I und 1 bis 99 Gew.-% wenigstens eines monoethylenisch ungesättigten und/oder konjugiert diethylenisch ungesättigten Comonomers b einpolymerisiert enthält, wobei die Comonomere b) ausgewählt sind unter
- monoethylenisch ungesättigten C₃-C₈-Mono- und C₄-C₈-Dicarbonsäuren,
- Amiden monoethylenisch ungesättigter C₃-C₈-Mono- oder C₄-C₈-Dicarbonsäuren,
- Anhydriden monoethylenisch ungesättigter C₄-C₈-Dicarbonsäuren,
- Hydroxy-C₂-C₄-alkylestern monoethylenisch ungesättigter C₃-C₈-Mono- oder C₄-C₈-Dicarbonsäuren,
- monoethylenisch ungesättigten Sulfonsäuren und deren Salze,
- monoethylenisch ungesättigten Nitrilen mit 3 bis 5 C-Atomen,
- N-Vinylheterocyclen,
- monoethylenisch ungesättigten Verbindungen mit wenigstens einer Poly-C₂-C₄-alkylenoxid-Gruppe,
- vinylaromatischen Kohlenwasserstoffen,
- Estern monoethylenisch ungesättigter C₃-C₈-Monocarbonsäuren mit C₁-C₂₀-Alkanolen, C₅-C₈-Cycloalkanolen, Phenyl-C₁-C₄-alkanolen oder Phenoxy-C₁-C₄-alkanolen,
- Diestern monoethylenisch ungesättigter C₄-C₈-Dicarbonsäuren mit C₁-C₂₀-Alkanolen, C₅-C₈-Cycloalkanolen, Phenyl-C₁-C₄-alkanolen oder Phenoxy-C₁-C₄-alkanolen,
- C₁-C₂₀-Alkylamiden und Di-C₁-C₂₀-alkylamiden monoethylenisch ungesättigter C₃-C₈-Monocarbonsäuren,
- Vinylestern aliphatischer Carbonsäuren mit 1 bis 20 C-Atomen,
- konjugiert diethylenisch ungesättigten C₄-C₁₀-Olefinen,
- C₂-C₂₀-Olefinen,
- halogensubstituierten C₂-C₂₀-Olefinen,
- monoethylenisch ungesättigten Monomeren, die eine oder zwei Epoxidgruppen aufweisen,
- monoethylenisch ungesättigten Monomeren, die wenigstens eine Carbonatgruppe aufweisen,
- Estern monoethylenisch ungesättigter C₃-C₈-Monocarbonsäuren mit C₈-C₂₄-Alkenolen oder C₈-C₂₄-Alkandienolen und Estern monoethylenisch ungesättigter Dicarbonsäuren mit C₈-C₂₄-Alkenolen oder C₈-C₂₄-Alkandienolen.

12. Polymer nach Anspruch 11, wobei das Polymer zusätzlich wenigstens ein Comonomer c einpolymerisiert enthält, das 2, 3 oder 4 nicht konjugierte, ethylenisch ungesättigte Doppelbindungen aufweist.

13. Verwendung von Polymeren nach einem der Ansprüche 8 bis 12 als Komponente in 2K-Bindemittelzusammensetzungen.

14. Verwendung nach Anspruch 13, wobei die Bindemittelzusammensetzung wenigstens eine Verbindung enthält, die wenigstens 2 funktionelle Gruppen F aufweist, welche unter aliphatischen Hydroxylgruppen, primären und sekundären Aminogruppen, Phosphingruppen, Phosphonatgruppen und Mercaptangruppen ausgewählt sind.

15. Verwendung nach Anspruch 14, wobei das Molverhältnis von funktionellen Gruppen der Formel I' zu den funktionellen Gruppen F im Bereich von 1 : 10 bis 10 : 1 liegt.

## Claims

1. A compound of the formula I 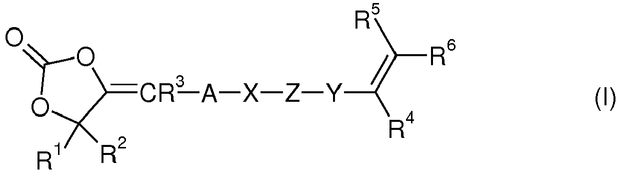 in which
R¹ and R² are each independently hydrogen, C₁-C₆-alkyl, C₁-C₄-alkoxy-C₁-C₄-alkyl, C₅-C₆-cycloalkyl, phenyl or phenyl-C₁-C₄-alkyl;
R³ is hydrogen, C₁-C₆-alkyl, C₁-C₄-alkoxy-C₁-C₄-alkyl, C₅-C₆-cycloalkyl, phenyl or phenyl-C₁-C₄-alkyl;
R⁴ is hydrogen, C₁-C₄-alkyl, CH₂COOR⁸, phenyl or phenyl-C₁-C₄-alkyl;
R⁵ and R⁶ are each independently hydrogen or C₁-C₄-alkyl, or one of the R⁵ and R⁶ radicals may also be COOR⁸ or CH₂COOR⁸;
A is a chemical bond or C₁-C₄-alkanediyl;
X is O or NR⁷;
Z is a chemical bond, PO₂, SO₂ or C=O;
Y is a chemical bond, CH₂ or CHCH₃;
R⁷, if present, is C₁-C₆-alkyl;
R⁸, if present, is hydrogen or C₁-C₆-alkyl.

2. A compound according to claim 1, in which R¹ and R² are each hydrogen or C₁-C₆-alkyl.

3. A compound according to either of the preceding claims, in which R³ is hydrogen.

4. A compound according to any of the preceding claims, in which
A is ethanediyl;
X is O; and
Z is C=O.

5. A compound according to any of the preceding claims, in which Y is a chemical bond.

6. A process for preparing compounds of the formula I, wherein a compound of the general formula II 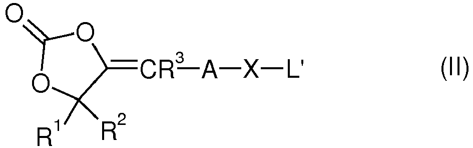 is reacted with a compound of the general formula III 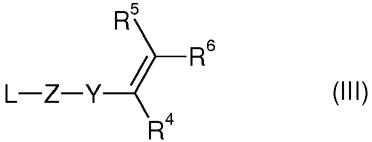 where
L is a nucleophilically displaceable leaving group;
L' is hydrogen or a C₁-C₄-alkylcarbonyl group
and A, X, Y, Z, R¹, R², R³, R⁴, R⁵ and R⁶ are each as defined in any of the preceding claims.

7. The process according to claim 6, wherein L is OH or C₁-C₈-alkoxy, Z is C=O and X is O, and wherein the reaction is performed under the conditions of an esterification or transesterification.

8. A polymer having a polymer backbone which is formed from carbon atoms and to which functional groups of the formula I' are attached 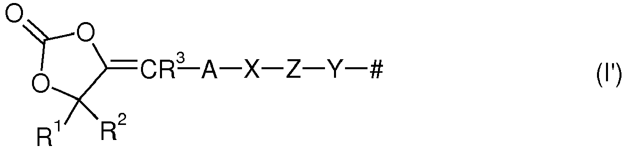 where # represents the bond to the polymer backbone and R¹, R², R³, A, X, Z and Y are each as defined in any of claims 1 to 5.

9. A polymer formed from polymerized ethylenically unsaturated compounds M, wherein the compounds M comprise at least 1% by weight, based on the total amount of the ethylenically unsaturated compounds forming the polymer, of at least one compound of the formula I according to claim 1.

10. A polymer according to either of claims 8 and 9 in the form of a homopolymer of the compound of the formula I.

11. A polymer according to either of claims 8 and 9 which comprises 1 to 99% by weight of at least one compound of the formula I and 1 to 99% by weight of at least one monoethylenically unsaturated and/or conjugatedly diethylenically unsaturated comonomer b in copolymerized form, wherein the comonomers b are selected from
- monoethylenically unsaturated C₃-C₈-mono- and C₄-C₈-dicarboxylic acids,
- amides of monoethylenically unsaturated C₃-C₈-mono- or C₄-C₈-dicarboxylic acids,
- anhydrides of monoethylenically unsaturated C₄-C₈-dicarboxylic acids,
- hydroxy-C₂-C₄-alkyl esters of monoethylenically unsaturated C₃-C₈-mono- or C₄-C₈-dicarboxylic acids,
- monoethylenically unsaturated sulfonic acids and salts thereof,
- monoethylenically unsaturated nitriles having 3 to 5 carbon atoms,
- N-vinylheterocycles,
- monoethylenically unsaturated compounds having at least one poly-C₂-C₄-alkylene oxide group,
- vinylaromatic hydrocarbons,
- esters of monoethylenically unsaturated C₃-C₈-monocarboxylic acids with C₁-C₂₀-alkanols, C₅-C₈-cycloalkanols, phenyl-C₁-C₄-alkanols or phenoxy-C₁-C₄-alkanols,
- diesters of monoethylenically unsaturated C₄-C₈-dicarboxylic acids with C₁-C₂₀-alkanols, C₅-C₈-cycloalkanols, phenyl-C₁-C₄-alkanols or phenoxy-C₁-C₄-alkanols,
- C₁-C₂₀-alkylamides and di-C₁-C₂₀-alkylamides of monoethylenically unsaturated C₃-C₈-monocarboxylic acids,
- vinyl esters of aliphatic carboxylic acids having 1 to 20 carbon atoms,
- conjugatedly diethylenically unsaturated C₄-C₁₀-olefins,
- C₂-C₂₀-olefins,
- halogen-substituted C₂-C₂₀-olefins,
- monoethylenically unsaturated monomers having one or two epoxide groups,
- monoethylenically unsaturated monomers having at least one carbonate group,
- esters of monoethylenically unsaturated C₃-C₈-monocarboxylic acids with C₈-C₂₄-alkenols or C₈-C₂₄-alkanedienols and esters of monoethylenically unsaturated dicarboxylic acids with C₈-C₂₄-alkenols or C₈-C₂₄-alkanedienols.

12. A polymer according to claim 11, wherein the polymer additionally comprises, in copolymerized form, at least one comonomer c having 2, 3 or 4 nonconjugated, ethylenically unsaturated double bonds.

13. The use of polymers according to any of claims 8 to 12 as a component in 2K binder compositions.

14. The use according to claim 13, wherein the binder composition comprises at least one compound having at least two functional groups F selected from aliphatic hydroxyl groups, primary and secondary amino groups, phosphine groups, phosphonate groups and mercaptan groups.

15. The use according to claim 14, wherein the molar ratio of functional groups of the formula I' to the functional groups F is in the range from 1:10 to 10:1.

## Revendications

1. Composés de formule I 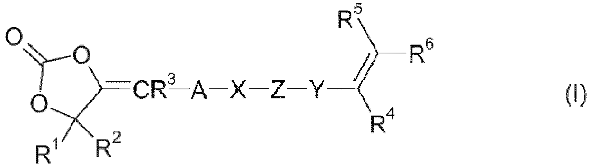 dans laquelle
R¹ et R² représentent indépendamment l'un de l'autre hydrogène, alkyle en C₁-C₆, alcoxy en C₁-C₄-alkyle en C₁-C₄, cycloalkyle en C₅-C₆, phényle ou phényl-alkyle en C₁-C₄ ;
R³ représente hydrogène, alkyle en C₁-C₆, alcoxy en C₁-C₄-alkyle en C₁-C₄, cycloalkyle en C₅-C₆, phényle ou phényl-alkyle en C₁-C₄ ;
R⁴ représente hydrogène, alkyle en C₁-C₄, CH₂COOR⁸, phényle ou phényl-alkyle en C₁-C₄ ;
R⁵ et R⁶ représentent indépendamment l'un de l'autre hydrogène ou alkyle en C₁-C₄, ou un des radicaux R⁵ ou R⁶ peut également représenter COOR⁸ ou CH₂COOR⁸ ;
A représente une liaison chimique ou alcanediyle en C₁-C₄ ;
X représente O ou NR⁷ ;
Z représente une liaison chimique, PO₂, SO₂ ou C=O ;
Y représente une liaison chimique, CH₂ ou CHCH₃ ;
R⁷ s'il est présent, représente alkyle en C₁-C₆ ;
R⁸ s'il est présent, représente hydrogène ou alkyle en C₁-C₆.

2. Composés selon la revendication 1, dans lesquels R¹ et R² représentent chacun hydrogène ou alkyle en C₁-C₆.

3. Composés selon l'une quelconque des revendications précédentes, dans lesquels R³ représente hydrogène.

4. Composés selon l'une quelconque des revendications précédentes, dans lesquels
A représente éthanediyle ;
X représente 0 ; et
Z représente C=O.

5. Composés selon l'une quelconque des revendications précédentes, dans lesquels Y représente une liaison chimique.

6. Procédé de fabrication de composés de formule I, dans lequel un composé de formule générale II 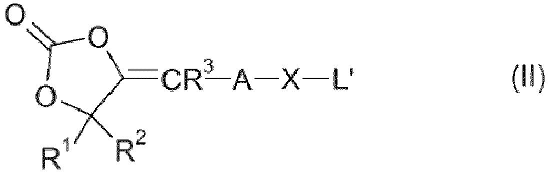 est mis en réaction avec un composé de formule générale III 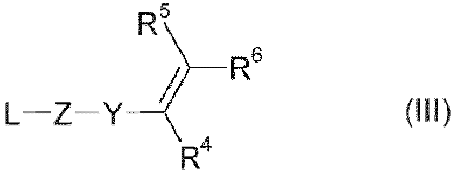
L représentant un groupe partant éliminable par voie nucléophile ;
L' représentant hydrogène ou un groupe alkylcarbonyle en C₁-C₄ ;
et A, X, Y, Z, R¹, R², R³, R⁴, R⁵ et R⁶ ayant les significations indiquées dans l'une quelconque des revendications précédentes.

7. Procédé selon la revendication 6, dans lequel L représente OH ou alcoxy en C₁-C₈, Z représente C=O et X représente 0, et dans lequel la réaction est réalisée dans les conditions d'une estérification ou d'une transestérification.

8. Polymère, qui comprend un squelette polymère constitué d'atomes de carbone, auquel des groupes fonctionnels de formule I' sont reliés 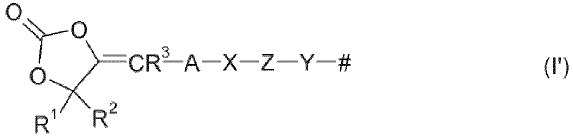 # représentant la liaison au squelette polymère, et R¹, R², R³, A, X, Z et Y ayant les significations indiquées dans l'une quelconque des revendications 1 à 5.

9. Polymère, qui est formé à partir de composés éthyléniquement insaturés M polymérisés, les composés M comprenant au moins 1 % en poids, par rapport à la quantité totale des composés éthyléniquement insaturés formant le polymère, d'au moins un composé de formule I selon la revendication 1.

10. Polymère selon l'une quelconque des revendications 8 ou 9, sous la forme d'un homopolymère du composé de formule I.

11. Polymère selon l'une quelconque des revendications 8 ou 9, qui contient 1 à 99 % en poids d'au moins un composé de formule I, et 1 à 99 % en poids d'au moins un comonomère b monoéthyléniquement insaturé et/ou diéthyléniquement insaturé conjugué, les comonomères b) étant choisis parmi
- les acides monocarboxyliques en C₃-C₈ et dicarboxyliques en C₄-C₈ monoéthyléniquement insaturés,
- les amides d'acides monocarboxyliques en C₃-C₈ ou dicarboxyliques en C₄-C₈ monoéthyléniquement insaturés,
- les anhydrides d'acides dicarboxyliques en C₄-C₈ monoéthyléniquement insaturés,
- les esters hydroxyalkyliques en C₂-C₄ d'acides monocarboxyliques en C₃-C₈ ou dicarboxyliques en C₄-C₈ monoéthyléniquement insaturés,
- les acides sulfoniques monoéthyléniquement insaturés et leurs sels,
- les nitriles monoéthyléniquement insaturés de 3 à 5 atomes C,
- les hétérocycles N-vinyle,
- les composés monoéthyléniquement insaturés contenant au moins un groupe poly-oxyde d'alkylène en C₂-C₄,
- les hydrocarbures aromatiques de vinyle,
- les esters d'acides monocarboxyliques en C₃-C₈ monoéthyléniquement insaturés avec des alcanols en C₁-C₂₀, des cycloalcanols en C₅-C₈, des phényl-alcanols en C₁-C₄ ou des phénoxy-alcanols en C₁-C₄,
- les diesters d'acides dicarboxyliques en C₄-C₈ monoéthyléniquement insaturés avec des alcanols en C₁-C₂₀, des cycloalcanols en C₅-C₈, des phényl-alcanols en C₁-C₄ ou des phénoxy-alcanols en C₁-C₄,
- les alkylamides en C₁-C₂₀ et les di-alkylamides en C₁-C₂₀ d'acides monocarboxyliques en C₃-C₈ monoéthyléniquement insaturés,
- les esters vinyliques d'acides carboxyliques aliphatiques de 1 à 20 atomes C,
- les oléfines en C₄-C₁₀ diéthyléniquement insaturées conjuguées,
- les oléfines en C₂-C₂₀,
- les oléfines en C₂-C₂₀ à substitution halogène,
- les monomères monoéthyléniquement insaturés qui comprennent un ou deux groupes époxyde,
- les monomères monoéthyléniquement insaturés qui comprennent au moins un groupe carbonate,
- les esters d'acides monocarboxyliques en C₃-C₈ monoéthyléniquement insaturés avec des alcénols en C₈-C₂₄ ou des alcanediénols en C₈-C₂₄ et les esters d'acides dicarboxyliques monoéthyléniquement insaturés avec des alcénols en C₈-C₂₄ ou des alcanediénols en C₈-C₂₄.

12. Polymère selon la revendication 11, dans lequel le polymère contient en outre au moins un comonomère c sous forme copolymérisée, qui comprend 2, 3 ou 4 doubles liaisons éthyléniquement insaturées non conjuguées.

13. Utilisation de polymères selon l'une quelconque des revendications 8 à 12 en tant que composant dans des compositions de liant 2K.

14. Utilisation selon la revendication 13, dans laquelle la composition de liant contient au moins un composé qui comprend au moins 2 groupes fonctionnels F, qui sont choisis parmi les groupes hydroxyle aliphatiques, les groupes amino primaires et secondaires, les groupes phosphine, les groupes phosphonate et les groupes mercaptan.

15. Utilisation selon la revendication 14, dans laquelle le rapport molaire entre les groupes fonctionnels de formule I' et les groupes fonctionnels F se situe dans la plage allant de 1:10 à 10:1.
